# EUROPEAN PATENT SPECIFICATION

(11) **EP 2 298 328 B1**
(45) Date of publication and mention of the grant of the patent: **16.04.2014**
(21) Application number: 10250970.0
(22) Date of filing: 24.05.2010
(51) Int. Cl.: A61K 35/44, A61P 25/28

(54) **Use of umbilical cord blood cells for the treatment of neurological disorders**
Verwendung von Nabelschnurzellen zur Behandlung von neurologischen Erkrankungen
Utilisation des cellules de sang de cordon ombilical pour le traitement de troubles neurologiques

(30) Priority: 25.05.2009 RU 2009119560; 31.07.2009 RU 2009129481
(43) Date of publication of application: 23.03.2011
(73) Proprietor: Cryocenter, Ltd., Moscow 117997 (RU)
(72) Inventor: Romanov, Yuriy Askoldovich, Moscow (RU); Smirnov, Vladimir Nikolaevich, Moscow (RU); Proshkin, Sergey Dmitrievich, Moscow (RU); Polyakov, Yuriy Izrailevich, Sankt-Petersburg (RU); Medvedev, Svyatoslav Vsevodolovich, Sankt-Petersburg, (RU); Paltsev, Mikhail Aleksandrovich, Moscow (RU); Sukhikh, Gennadiy Tikhonovich, Moscow (RU); Savchenko, Valeriy Grigorievich, Moscow (RU); Radaev, Sergey Maksimovich, Moscow (RU); Klimov, Igor Aleksandrovich, Moscow (RU); Gavrilova, Svetlana Ivanovna, Moscow (RU); Boiko, Aleksey Nikolaevich, Moscow (RU); Davydovskaya, Maria Vafaevna, Moscow (RU); Smulevich, Anatoliy Boleslavovich, Moscow (RU); Dubnitskaya, Etheri Broneslavovna, Moscow (RU); Pavlova, Lyubov Konstantinovna, Moscow (RU); Morozova, Yana Vyacheslavovna, Serpuhov, Moscow region (RU); Khachatrian, William Aranovich, Sankt-Petersburg (RU); Lebedev, Konstantin Eduardovich, Sankt-Petersburg (RU)
(74) Representative: Woods, Geoffrey Corlett

(56) References cited:
- WO-A1-2005/059115
- WO-A2-2009/023814
- KURTZBERG JOANNE: "Update on umbilical cord blood transplantation.", CURRENT OPINION IN PEDIATRICS FEB 2009 LNKD- PUBMED:19253461, vol. 21, no. 1, February 2009 (2009-02), pages 22-29, XP002617394, ISSN: 1531-698X
- ESCOLAR MARIA L ET AL: "Transplantation of umbilical-cord blood in babies with infantile Krabbe's disease.", THE NEW ENGLAND JOURNAL OF MEDICINE 19 MAY 2005 LNKD- PUBMED:15901860, vol. 352, no. 20, 19 May 2005 (2005-05-19) , pages 2069-2081, XP002617395, ISSN: 1533-4406
- BEAM ET AL: "Outcomes of Unrelated Umbilical Cord Blood Transplantation for X-Linked Adrenoleukodystrophy", BIOLOGY OF BLOOD AND MARROW TRANSPLANTATION, KLUGE CARDEN JENNINGS PUBLISHING, CHARLOTTESVILLE, VA, US, vol. 13, no. 6, 22 May 2007 (2007-05-22), pages 665-674, XP022089701, ISSN: 1083-8791, DOI: DOI:10.1016/J.BBMT.2007.01.082
- NEWMAN M B ET AL: "TRANSPLANTATION OF HUMAN UMBILICAL CORD BLOOD CELLS IN THE REPAIR OF CNS DISEASES", EXPERT OPINION ON BIOLOGICAL THERAPY, ASHLEY, LONDON, GB, vol. 4, no. 2, 1 February 2004 (2004-02-01), pages 121-130, XP009037136, ISSN: 1471-2598, DOI: DOI:10.1517/14712598.4.2.121
- GARBUZOVA-DAVIS SVITLANA ET AL: "Intravenous administration of human umbilical cord blood cells in an animal model of MPS III B.", THE JOURNAL OF COMPARATIVE NEUROLOGY 1 JUL 2009 LNKD- PUBMED:19399896, vol. 515, no. 1, 20 March 2009 (2009-03-20), pages 93-101, XP002617396, ISSN: 1096-9861
- MCGOWAN E ET AL: "A decade of modeling Alzheimer's disease in transgenic mice", TRENDS IN GENETICS, ELSEVIER SCIENCE PUBLISHERS B.V. AMSTERDAM, NL, vol. 22, no. 5, 1 May 2006 (2006-05-01), pages 281-289, XP028054929, ISSN: 0168-9525, DOI: 10.1016/J.TIG.2006.03.007 [retrieved on 2006-05-01]

## Description

### Field of the Invention

The invention relates to medicine and deals with using umbilical cord blood cells for treatment of mental and neurological disorders.

### Description of the Prior Art

The umbilical cord blood collected at birth represents a unique source of pluripotent stem cells [Atala et al. Umbilical Cord Blood. Current Stem Cell Research &Therapy, 2007, Vol.2, No.4 307].

The uniqueness of umbilical cord blood cells consist in that these postnatal stem cells are the only ones capable of supporting hematopoiesis and creating a complete human immune system, when transplanted to experimental animals, giving rise to B- and T-lymphocytes and dendritic cells, primary and secondary lymphoid organs and inducing functional human immune responses [Traggai E., Chicha L., Mazzuchelli et al. Development of human adaptive immune system in cord blood cell-transplanted mice. Science 2004; 304:14-107].

Hematopoietic stem cells (CD34+) and endothelial progenitor cells (CD133+) are the most prevalent stem cell populations found in umbilical cord blood. A high level of hematopoietic cells-progenitors enables to efficiently use the transplantation of umbilical cord blood cells as an alternative to bone marrow transplantation in acute and chronic leukemia, lymphomas, Fanconi anemia and other hematological diseases [Barker J. Umbilical cord blood (UCB) transplantation: An alternative to the use of unrelated volunteer donors? Hematology Am Soc Hematol Educ Program. 2007: 55-61].

Apart from hematopoietic, umbilical cord blood contains other types of stem cells, including embryonic-like cells, epithelial precursor cells, mesenchymal stem cells and unrestricted somatic stem cells [Harris David T., Rogers I. Umbilical cord blood: A Unique Source of Pluripotent Stem Cells for Regenerative Medicine, Current Stem Cell Research & Therapy, 2007, 2, 301-309].

Pluripotent properties of umbilical cord blood stem cells have been demonstrated in a whole series of in vitro and in vivo studies. Under culture conditions, umbilical cord blood stem cells are able to differentiate into osteoblasts, chondroblasts, adipocytes and nerve cells as well as into endothelial and muscular cells, hepatocytes and insulin-producing cells [Kogler G., Sensken S.& Wernet P. (2006) Comparative generation and characterization of pluripotent unrestricted somatic stem cells with mesenchymal stem cells from human cord blood. Experimental hematology, 34(11), 1589-95 and Tondreau T., Meuleman N., Delforge A. et al. Mesenchymal stem cells derived from CD133-positive cells in mobilized blood and cord blood: proliferation and plasticity. Stem Cells, 2005; 23:1105-1112].

Umbilical cord blood stem cells are capable of homing and when transplanted to experimental animals they can ameliorate the course of a number of diseases, including diabetes, cardiovascular and neurological and ophthalmologic diseases, the locomotor system diseases, etc.

Within the whole life span, a human is permanently at risk of getting neurological damages and traumas. Thus, cerebral palsy occurs in high rates in newborns. Both children and adults suffer from spinal cord injuries. The risk of embolic and hemorrhagic strokes increases among those attained 40 years of age. And, finally in late middle (40 years and upwards) and old age the individuals face the prospect of neurodegenerative diseases such as Parkinson's disease [Harris D.T., Badowski M., Ahmad N. & Gaballa M. (2008). The potential of cord blood stem cells for use in regenerative medicine. Expert Opinion on Biological Therapy, 7(9), 1311-1322 and Harris D.T. & Rogers I. Umbilical cord blood: a unique source of pluripotent stem cells for regenerative medicine. Current Stem Cell Research & Therapy, 2, 301-309]. Traditional methods of therapy applied in relation to neurological disorders and injuries are inefficient what places a burden on society. The use of stem cells for neurological disorders or injuries would open the possibility of replacement or regeneration of damaged tissues of the spinal cord or the brain.

In animal models with neurological damages due to brain injuries, the infusion of umbilical cord blood cells has resulted in improved state [Chen J., Sanberg P.R., Li Y. et al. Intravenous administration of human umbilical cord blood reduces behavioral deficits after stroke in rats. Stroke 2001; 32: 2682-2688; Willing A.E., Lixian J., Milliken M. et al. Intravenous versus intrastriatal cord blood administration in a rodent model of stroke. J Neurosci Res 2003; 73(3); 296-307; Borlongan C.V., Hadman M., Sanberg C.D. Central Nervous System Entry of Peripherally Injected Umbilical Cord Blood Cells Is Not Required for Neuroprotection in Stroke. Stroke 2004; 35: 2385-2389; Newman M.B., Willing A.E., Manressa J.J., Sanberg C.D., Sanberg P.R. Cytokines produced by cultured human umbilical cord blood (HUCB) cells: implications for brain repair. Exp Neurol 2006, 199(1): 201-208; Vendrame M., Cassady J., Newcomb J. et al. Infusion of human umbilical cord blood cells in a rat model of stroke dose dependently rescues behavioral deficits and reduces infarct volume. Stroke 2004; 35: 2390-2395; Xiao J., Nan Z., Motooka Y., Low W.C. Transplantation of a novel cell line population of umbilical cord blood stem cells ameliorates neurological deficits associated with ischemic brain injury. Stem Cells Dev 2005; 14: 722-733; Newcomb J.D., Ajrno C.T., Sandberg C.D. et al. Timing of Cord Blood Treatment After Experimental Stroke Determines Therapeutic Efficacy. Cell Transplant 2006; 15: 213-223; Nan Z., Grande A., Sanberg C.D., Sanberg P.R., Low W.C. Infusion of Human Umbilical Cord Blood Ameliorates Neurologic Deficits in Rats with Hemorrhagic Brain Injury. Ann NY Acad Sci 2005, 1049(1): 84-96; Bliss T., Guzman R., Daadi M., Steinberg G.K. Cell transplantation therapy for stroke. Stroke 2007; 38: 817-826; Saporta S., Kim J.J., Willing W.E. et al. Human umbilical cord blood stem cells infusion in spinal cord injury: engraftment and beneficial influence on behavior. J Hematother Stem Cell Res 2003; 12: 271-278; Kuh S.U., Cho Y.E., Yoon D.H. et al. Functional recovery after human umbilical cord blood cells transplantation with brain-derived neurotropic factor into the spinal cord injure rats. Acta Neurochir (Wein) 2005; 14: 985-992; Kang K.S., Kim S.W., Oh Y.H. et al. Thirty-seven-year old spinal cord-injured female patient, transplanted of multipotent stem cells from human UC blood with improved sensory perception and mobility, both functionally and morphologically: A case study. Cytotherapy 2005; 7: 368-373; Lu D., Sanberg P.R., Mahmood A. et al. Intravenous administration of human umbilical cord blood reduces neurological deficit in the rat after traumatic brain injury. Cell Transplant 2002; 11: 275-281; Meier C., Middleanis J., Wasielewski B. et al. Spastic paresis after perinatal brain damage in rats is reduced by human cord blood mononuclear cells. Ped Res 2006; 59: 244-249; Ende N., Chen R. Parkinson's Disease Mice and Human Umbilical Cord Blood. J Med 2002; 33: 173-80; Garbuzova-Davis S., Willing A.E., Zigova T. et al. Intravenous administration of human umbilical cord blood stem cells in a mouse model of amyotrophic lateral sclerosis: distribution, migration, and differentiation. J Hematother Stem Cell Res 2003; 12: 255-270].

Jang and colleagues [Jang Y.K., Park J.J., Lee M.C. et al. Retinoic acid-mediated induction of neurons and glial cells from human umbilical cord-derived hematopoietic stem cells. J Neurosci Res 2004; 75: 573-584] have demonstrated that CD133+ umbilical cord blood stem cells under the influence of retinoid acid differentiate into neuronal (astrocytes and oligodendrocytes) and glial cells with neuromarkers, including tubulin, neuron-specific enolase, NeuN, microtubulin-associated protein-2 (MAP2) and glial fibrillar acidic protein (GFAP) that is an astrocyte-specific marker.

Moreover, non-hematopoietic stem cells found in umbilical cord blood are able to differentiate into astrocytes and oligodendrocytes [Buzanska L., Jurga M., Stachowiak E.K., Stachowiak M.K., Domanska-Janic K. Neural stem-like cell line derived from a non-hematopoietic population of human umbilical cord blood. Stem Cells Develop 2006; 15: 391-406]. Therefore, the progeny of non-hematopoietic umbilical cord blood stem cells could be useful as well in treatment of brain injuries and neurological disorders.

In a spinal cord injury model in rats the infusion of umbilical cord blood stem cells resulted in significant improvements on day 5 post-infusion compared to control animals. Stem cells were identified in the regions of nerve tissue injuries, but were not found in uninjured regions of the spinal cord. [Saporta S., Kim J.J., Willing A.E. et al. Human umbilical cord blood stem cells infusion in spinal cord injury: engraftment and beneficial influence on behavior. J Hematother Stem Cells Res 2003; 12: 271-278]. These findings complement the results of investigations on the ability of umbilical cord blood cells transplanted into spinal cord of animals with injuries to differentiate into varied kinds of nerve cells and to improve regeneration of axons and motor function [Kang K.S., Kim S.W., Oh Y.H. et al. Thirty-seven-year old spinal cord-injured female patient, transplanted of multipotent stem cells from human UC blood with improved sensory perception and mobility, both functionally and morphologically: A case study. Cytotherapy 2005; 7: 368-373].

As early as 2001, using the most prevalent stroke model (medial carotid artery occlusion) Cheng J. and colleagues demonstrated that the infusion of umbilical cord blood cells into rats could ameliorate various physical and behavioral disease-related deficits [Chen J., Sanberg P.R., Li Y. et al. Intravenous administration of human umbilical cord blood reduces behavioral deficits after stroke in rats. Stroke 2001; 32: 2682-2688].

The studies demonstrate that direct injection of stem cells into the brain is not required [Willing A.E., Lixian J., Milliken M. et al. Intravenous versus intrastriatal cord blood administration in a rodent model of stroke. J Neurosci Res 2003; 73(30): 296-307]. As a matter of fact, beneficial effects can be observed even if stem cells do not reach the target organ (possibly via the release of growth and repair factors triggered by anoxia) [Borlongan C.V., Hadman M., Sanberg C.D. Central Nervous System Entry of Peripherally Injected Umbilical Cord Blood Cells Is Not Required for Neuroprotection in Stroke. Stroke 2004; 35: 2385-2389 and Newman M.B., Willing A.E., Manressa J.J., Sanberg C.D., Sanberg P.R. Cytokines produced by cultured human umbilical cord blood (HUCB) cells: implications for brain repair. Exp Neurol 2006, 199(1): 201-208].

It should be especially noted that unlike current pharmacological intervention that requires application in first few hours after stroke, therapy using umbilical cord blood cells transplanted up to 48 h after a thrombotic event is still effective [Newcomb J.D., Ajrno C.T., Sandberg C.D. et al. Timing of Cord Blood Treatment After Experimental Stroke Determines Therapeutic Efficacy. Cell Transplant 2006; 15: 213-223]. The advantages of therapy using umbilical cord blood cells were also shown in a model of hemorrhagic stroke [Nan Z., Grande A., Sanberg C.D., Sanberg P.R., Low W.C. Infusion of Human Umbilical Cord Blood Ameliorates Neurologic Deficits in Rats with Hemorrhagic Brain Injury. Ann NY Acad Sci 2005, 1049(1): 84-96].

The use of umbilical cord blood mononuclear cells in treatment of coronary heart disease, myocardial infarction, cerebral ischemia induced by atherosclerosis or acute cerebral circulation disorder is described in Patent RU 2 284 190. The disadvantage of the method includes the mandatory HLA-typing and, moreover, to achieve a clinical effect in ischemic states the donor's umbilical cord blood cells must differ from the recipient's cells, at least, in four antigens out of a set of HLA-A-A, HLA-B, HLA-DR antigens.

Apart from strokes, the presumable usage of umbilical cord blood cells in treatment of other forms of neurological damages is demonstrated in animal models. The studies [Lu et al. Intravenous administration of human umbilical cord blood reduces neurological deficit in the rat after traumatic brain injury. Cell Transplant 2002; 11: 275-281 have shown in a rat model that intravenous infusion of umbilical cord blood mononuclear cells could be useful in treatment of traumatic brain injuries. In this model the umbilical cord blood cells were observed to enter the brain, selectively migrate to the damaged region of the brain, express neuronal markers, and reduce the neurological damage.

Similarly, umbilical cord blood cells graft could also improve symptoms of newborn cerebral palsy in a rat model [Meier C., Middleanis J., Wasielewski B. et al. Spastic paresis after perinatal brain damage in rats is reduced by human cord blood mononuclear cells. Ped Res 2006; 59: 244-249].

Focusing on the functional regeneration of damaged tissues, regenerative therapy using umbilical cord blood stem cells is especially urgent for diseases of the central nervous system, in particular, degenerative diseases and posttraumatic encephalopathy.

On the one hand, the occurrence of traumatic brain injuries is rather high (the traumatic brain injury is recorded in 4 persons out of 1000 of the population), on the other hand, in patients that suffered moderate traumatic brain injury the signs of posttraumatic psychoorganic syndrome, asthenization, headaches, vegetovascular dysfunction, static or coordination disturbances and other neurological symptoms remain frequently even after a prolonged period (Lichterman L.B., Fraerman A.P., Khitrin L.Kh. 1979, Staging of clinical course of craniocerebral injury, Gorky, 1979, pp. 168-178; E.N.Kligunenko, L.A.Dzyak, V.I.Grishin, O.A.Zozulya, 2004; Dmitrieva A.S., Klimenko T.V., 1998).

However, clinical studies to investigate the effectiveness of therapy using umbilical cord blood cells for the consequences of traumatic brain injuries have not been conducted. It should be noted that an experimental model of traumatic brain injury in animals may significantly differ from the real clinical situation, which leads to more pronounced brain lesions.

Alzheimer's disease is the most prevalent cause of dementia developing in the middle- and old-age. The main role in pathogenesis of the primary neurodegenerative alzheimer process is considered to be played by impaired conversions of the beta-amyloid precursor protein (A13), accompanied by the accumulation of insoluble All in the cerebral parenchyma in the form of aggregated accumulations - amyloid plaques. In their turn, the A13 aggregates are the potential activators of microglia and astrocytes which respond to cerebral amyloidosis by activating chronic inflammatory process [Benzing W.C., Wujek J.R., Ward E.K., Shaffer D., Ashe K.N. et al. Evidence for glia-mediated inflammation in aged APP(SW) transgenic mice. Neurobiol Aging, 1999, 20, p.581-589].

Although, it has been earlier considered that the activation of microglia and astrocytes presents only an epiphenomenon and does not take part in pathogenesis of Alzheimer's disease, recent studies have revealed that the Aβ-induced inflammatory cascade is an important pathogenetic mechanism of an Alzheimer-type neurodegenerative process. It has been proved that therapeutic strategies targeted to various components of this inflammatory cascade, including non-steroidal anti-inflammatory drugs, immunization and modulating of microglial activation reduce Alzheimer-like beta-amyloid pathology, cognitive deficit and behavioral disorders in an Alzheimer's disease model in transgenic mice, and in some cases even reduce pathology in patients with Alzheimer's disease [Bard F., Cannon C., Barbour R., Burke R.L., Games D et al. Peripherally transplanted antibodies against amyloid-beta-peptide enter the central nervous system and reduce pathology in a mouse model of Alzheimer disease. Nature Med., 2000, 6, p. 916-919; Lim G.P., Chu T., Yang F., Beech W., Frautschy S.A. et al. The curry spice curcumin reduces oxidative damage and amyloid pathology in an Alzheimer transgenic mouse. J.Neurosci, 2001, 21, p. 8370-8377; Nicoll J.A., Wilkinson D., Holmes C., Steart P., Markham H. et al. Neuropathology of human Alzheimer disease after immunization with amyloid-beta-peptide: a case report. Nature Med., 2003, 9, p. 448-452; Schenk D., Barbour R., Dunn W., Gordon G., Grajeda H. et al. Immunization with amyloid-beta attenuates Alzheimer-disease-like pathology in the PDAPP mouse. Nature, 1999, 400, p. 173-177; Szekely C.A., Thorne J.E., Zandi P.P., Ek M., Messias E. Nonsteroidal anti-inflammatory drugs for the prevention of Alzheimer's disease: a systematic review. Neuroepidemiology, 2004, 23, p.159-169; Veld B.A., Ruitenberg A., Hofman A., Launer L.J., van Duijn C.M. Nonsteroidal anti-inflammatory drugs and the risk of Alzheimer's disease. N.Engl. J. Med., 2001, 345, p.1515-1521].

As has been shown in animal models, the human umbilical cord blood cells augment an anti-inflammatory response of type 2 T-helpers, and this response was associated with reduced infarct volume in brain tissue [Vendrame M., Cassady J., Newcomb J., Butler T., Pennypacker K.R. et al. Infusion of human umbilical cord blood cells in a rat model of stroke dose-dependently rescues behavioral deficits and reduces infarct volume. Stroke, 2004, 35, p. 2390-2395]. Later on this therapeutic effect was confirmed in other models of neuro-inflammatory processes as well [El-Badri N.S., Hakki A., Saporta S., Liang X., Madhusodanan S. et al. Cord for glial-mediated inflammation in aged APP(SW) transgenic mice. Neurobiol blood mesenchymal stem cells: Potential use in neurological disorders. Stem Cells Dev., 2006, 15, p. 497-506; Garbuzova-Davis S., Gografe S.J., Sanberg C.D., Willing A.E., Saporta S. et al. Maternal transplantation of human umbilical cord blood cells provides prenatal therapy in Sanfilippo type B mouse model. FASEB J., 2006, 20, p.485-487; Henning R.J., Burgos J.D., Ondrovic L., Sandberg P., Balis J. et al. Human umbilical cord blood progenitor cells are attracted to infarcted myocardium and significantly reduce myocardial infarction size. Cell Transplant., 2006, 15, p.647-658].

In experiments conducted in an Alzheimer-disease model in animals, this effect was associated with an increased life span of transgenic mice [Nikolic W.V., Hou H., Town T., Zhu Y., Giunta B. Peripherally transplanted human umbilical cord blood cells reduce parenchymal and vascular 13-amyloid deposits in Alzheimer mice. Stem Cells Dev., 2008, 17, p. 1-17]. The researches have reported on a significant reduction of the A β level and the number of amyloid plaques in transgenic mice after a series of i.v. infusions of the umbilical cord blood stem cells concentrate.

Parkinson's disease, which incidence rate in the middle-aged persons is also high, presents a neurodegenerative disease with a "dotted" locus - the substantia nigra cells damage leading to their death. Although this pathology has been studied for almost two centuries, etiology and genesis of the process still require credible evidence. The hypotheses on an exotoxic role of some substances (for instance, under the MAO B effect 1-methyl-4-phenyl-1,2,3,6-tetrohydroperidin converses into N-methyl-4-phenylpyridinum compound toxic for substantia nigra neurons) or a genetic factor (a relation with segment 4q21-q23 with autosomal dominant inheritance) still remain assumptions [Cleeter MWJ et al.: Irreversible inhibition of mitochondrial complex I by 1-methyl-4-phenylpyridinum: Evidence for free radical involvement. J. Neurochem 58:786, 1992; Polymeropoulos MH et al: Mapping of a gene for Parkinson's disease to chromosome 4q21-q23. Science 274: 1197, 1996].

The deficit of dophamin-synthesizing function due to the death of the above cells leads to the failure of a mediator chain of the extrapyramidal system resulting in motor cortex inhibition and clinical signs and symptoms of Parkinson's disease. This problem requires also an urgent investigation owing to epidemiological data (the prevalence of Parkinson's disease among the population as a whole amounts to 1-2 in 1000, and among persons over 65 years already every hundredth suffers from this disease).

Presently used methods of medication therapy (drugs containing L-DOPA; agonists of D2/D3 dophamin receptors; MAO inhibitors, etc.) are inefficient as they lead to progression of the process and present a symptomatic approach with multiple limitations (Aminoff M.J.: Treatment of Parkinson's disease. West J Med 161: 303, 1994). Furthermore, administration of this therapy is time-limited. Thus, the restoration of normal natural production of dophamin by resuscitated (or newly grown) cells of substantia nigra presents a key problem in the treatment of Parkinson's disease. Taking into account a degenerative nature of the pathological process in Parkinson's disease, the implementation of cell intervention targeted to restore the devastated area of the nervous tissue presents the most attractive approach.

For lateral amyotrophic sclerosis, Parkinson's disease in animal models, the infusion of umbilical cord blood cells resulted in behavioral improvements compared to control animals [Ende N., Chen R. Parkinson's Disease Mice and Human Umbilical Cord Blood. J Med 2002; 33: 173-80; Garbuzova-Davis S., Willing A.E., Zigova T. et al. Intravenous administration of human umbilical cord blood stem cells in a mouse model of amyotrophic lateral sclerosis: distribution, migration, and differentiation. J Hematother Stem Cell Res 2003; 12: 255-270].

A method for treatment of lateral amyotrophic sclerosis by infusion into blood of umbilical cord blood nucleated cells in the quantity not less than 5 x 10⁹ is known (WO 2004/071283). The implementation of the method described in WO 2004/071283 does not require preliminary suppression of immune response or HLA-typing in the donor or recipient, however, extremely large amounts of cells are needed for its implementation. The number of nucleated cells in one portion of umbilical cord blood does not exceed 2.8x10⁹. This amount is insufficient to implement the method. Therefore, it is required to administer a single-step infusion of allogeneic cells and the cells harvested from several different donors. In this case, the risk of allergic, including anaphylactic reactions, and the risk of development of other immunopathological states, including immune depression, sharply increases.

Apart from that, a single-step immunization using cells from different donors makes it impossible to apply this method repeatedly in the same patient. The application of the method has resulted in toxic and allergic reactions in 5 out of 10 cases with immune suppression developed in all patients within 7-30 days post-infusion. An attempt of repeated application of the method has resulted in toxico-allergic reactions in the majority of patients.

The problem of treatment of multiple sclerosis, chronic demyelinating disease of the central nervous system (CNS), remains one of the most urgent issues in current neurology owing to a high prevalence of this disease among young employable persons and inevitable development of stable disability at a certain stage.

Identification of various types of demyelination - from predominant inflammatory reactions to oligodendrogliopathy and abnormal remyelination - as well as evidences of axonal damage not only in active foci, but also in externally unchanged substantia alba have brought disseminated sclerosis closer to neurodegenerative diseases (Lucchinetti C. et al. Brain Pathol., 1996, V.6, P. 25922; Trapp B.D. et al. Neuroscience, 1999,V.5, p.48].

Immunopathological mechanisms of acute states developing in multiple sclerosis [Zavalishin I.A., Zakharova M.N. Disseminated sclerosis and other demyelinating diseases. Edited by Guseva E.I, et al. M., 2004. P.60; Disseminated sclerosis. Selected issues of theory and practice. Edited by Zavalishina I.A., Golovkina V.I. M., 2000; Hohlfeld R. Brain, 1997, V.120, P.865] include the activation of energetic, inactive type CD4+ T cells outside the CNS; their penetration through the hematoencephalic barrier (HEB); the formation of a trimolecular complex including the corresponding receptor of the activated T cell and autoantigen connected with molecules class II of the major histocompatibility complex on antigen-presenting cells (the latter includes macrophages and gliacytes). Having penetrated into the nervous system, autoreactive T cells and repeatedly activated macrophages and microglial cells secrete anti-inflammatory cytokines (interferon-gamma, tumor necrosis factor-alpha, lymphotoxin, etc.), which in their turn in a greater degree induce and support inflammatory reactions and augment permeability disorders in the hematoencephalic barrier. Although at initial stages the clinical course of multiple sclerosis is characterized by alternation between aggravation and remission, the immuno-inflammatory process as a whole is characterized by a permanent course.

Damage to myelin and oligodendrocytes is developing as a result of a cascade of immunological and biochemical disorders. At later stages of the pathological process, nonspecific mechanisms, i.e., phagocytosis of damaged structures and proliferation of glial elements are activated. However, aside from the development of inflammatory reactions, demyelinating process and glial disorders, the main role in the progression of disseminated sclerosis is played by the recruitment of axons into the process. It is the axonal damage in multiple sclerosis that is considered to be responsible for development of irreversible neurological deficit and transformation of a remitting course with lability of symptoms into a secondary progressive course [Trapp B.D. et al. Neuroscience, 1999, V. 5, P.48].

The most probable causes of axonal destruction in multiple sclerosis are considered. The mechanism of direct immunological attack of axons in multiple sclerosis is presumed to be the expression of the molecule class I of the major histocompatibility complex on them resulting in their vulnerability to cytotoxic impact of CD8+ T lymphocytes [Neumann H. et al. Science, 1995, V.269, P. 549]. The development of axonal degeneration owing to inflammatory reactions may be associated with various factors [Rieckmann P., Maurer M., Curr. Opin. Neurol., 2002, V.15, p.361], including an intracellular pressure rise in inflammatory edema; the damage as a result of glutamate (produced by activated macrophages and microglia) impact upon oligodendroglial AMPA-receptors; the release of pro-inflammatory cytokines.

However, degenerative changes of axons are possible also in the chronic demyelinating process at the expense of disordered myelin-axonal interactions and trophic influence of oligodendrogliocytes, in particular, a modulating impact of myelin-associated glycoprotein and lipoprotein. Mechanisms of their trophic influence upon the axon are diverse. However, in deficit of both said proteins, axonal pathology is developing in a nodal region with axonal cytoskeleton being damaged [Trapp B.D. et al. Neuroscience, 1999, V.5, p 48].

Another important aspect of axonal-glial interactions is the impact of trophic factors. Thus, in a group of patients suffering from disseminated sclerosis with an early onset and severe course of disease, mutations have been identified leading to the lack of ciliary neurotrophic factor [Ciess R. et al. Arch. Neurol., 2002, V. 59, p.407]. A similar effect on the course of disease is described also in patients with apolipoprotein E e4 allel, in whom early onset, higher rate of aggravations and more rapid progression of disease are observed [Fazekas E. et al. Neurology, 2001, V.57, p. 853]. These data suggest that irrespective of immune-mediated damage to myelin and axons, genetically determined structural and molecular integrity of axonal-glial interactions is critical in a demyelinating process.

The development of neurological deficit in multiple sclerosis is considered from a viewpoint of two pathogenetic mechanisms: reversible acute inflammatory demyelination dominant in an acute stage of disease with a remitting course of multiple sclerosis and irreversible axonal degeneration prevalent in case of a secondary progressive course [Trapp B.D. et al. Neuroscience, 1999, V.5, p 48].

These same changes also govern the development of atrophic processes in the CNS in multiple sclerosis. Firstly, demyelination per se leads to the loss of compact myelin and, consequently, brain parenchyma. Secondly, as a result of axonal damage secondary axonal degeneration develops in acute foci of demyelination.

A primary progressive course of multiple sclerosis occupies a special place owing to its clinical, genetic, immunobiochemical, neuro-optical and morphological distinctions from remitting and secondary progressive variants [Ge Y. et al. Radiology, 2000, V. 214, P.665; Redmond I.T. et al. Acta Neurol Scand, 2000, V. 102, P.99], what enables to presume the presence of specific pathogenetic mechanisms in the primary-progressive course of multiple sclerosis. They include primary damage to oligodendrogliocytes, secondary demyelination with remyelination and signs of inflammation being absent, as well as diffuse axonal pathology, which could precondition a gradual growth of neurological deficit from the very onset of disease.

At present a standard scheme in therapy of multiple sclerosis includes the use of glucocorticosteroids (intravenous pulse-therapy using methylprednisolon, tabletted methylprednisolon), which positively influence the rate of recovery from acute neurological disorders [Goodin D.S. et al. Neurology, 2002, V.58, p 169]. Furthermore, pulse-therapy is used also in an aggravated state at the background of secondary progression of neurological symptoms. In the primary progressing course of multiple sclerosis it, as a rule, is ineffective. To arrest severe exacerbations of multiple sclerosis in pronounced neurological deficit, especially in disorder of vital functions, plasmapheresis may be used in combination with intravenous infusion of methylprednisolon.

To prevent or decrease the incidence rate of exacerbations, stabilize the state, prevent transformation into the progressive course (in remitting multiple sclerosis), and to slow down disability snowballing in the secondary progradient course, interferons (betaferon and rebif for subcutaneous injection, avonex for intramuscular injection) and glatiramer acetate (Copaxone-Teva) are prescribed as immunomodulating preparations.

Side effects in interferon treatment include, inter alia, flu-like (fever, headaches, myalgia and arthralgia), local (hyperemia, painfulness), cardiovascular (hypotension, tachycardia and arrhythmias) and hematological (leucopenia, thrombocytopenia) reactions; changes in hepatic function (a raised level of bilirubin, transaminase); neurological symptoms (an increase in spasticity, and more infrequently an aggravation of other neurological symptomatology at the background of flu-like phenomena). The most frequent side effects of glatiramer acetate treatment include local reactions, however, general systemic side effects, including clinical manifestations of vasodilatation, chest pain, dyspnea, palpitations, and anxiety can also develop.

Traditional therapy for both remitting and secondary progressive multiple sclerosis should be administered as a continuous treatment regimen. Furthermore, taking into account immunogenicity and side effects of each preparation modulating the course of disseminated sclerosis, when prescribing this treatment, an individual approach is required.

Lately, the results of trials of IgG intravenous infusion in limited groups of patients have been published. They suggest that the rate of exacerbations in a remitting course of disease may be decreased. However, at present the main problems in IgG application for multiple sclerosis include the need to conduct clinical investigations and the lack of a clearly defined administration scheme at a high cost of the preparation.

To raise the efficiency of treatment of schizophrenia patients is among the most urgent tasks of current psychiatry owing to severe consequences of the disease as well as to problematicity and discussibility of principal probability of retrogressive reduction of a part of symptomatology with compensation of some lost functions and, finally, to pharmacogenetic deficit caused by side effects of neuroleptic therapy.

Current psychiatry identifies parameters of cognitive functions, which study has received the status of an independent scientific school, as a significant feature of remissions in schizophrenia [Green M.F. What are the functional consequences of neurocognitive deficits in schizophrenia? Am J Psychiat, 1996, Vol. 153, p.p. 321-330; Green M.F., Olivier B., Crawley J.N., Penn D.L., Silverstein S. Social Cognition in Schizophrenia: Recommendations from the Measurement and Treatment Research to Improve Cognition in Schizophrenia New Approaches Conference. Schizophr. Bull. Oct. 2005, vol. 31, p.p.882-887; Green M.F., Penn D.L., Bentall R., Carpenter W.T., Gaebel W., Gur R.C., Kring A.M., Park S., Silverstein S.M., Heinssen R. Social Cognition in Schizophrenia: An NIMH Workshop on Definitions, Assessment, and Research Opportunities. Schizophr. Bull. Nov. 2008, vol.34, p.p.1211-1220].

The signs of decreased cognitive functioning in schizophrenia patients observed at least 10 times more often than in healthy persons (85-94% versus 7%, respectively) [Saykin A.J., Shtasel D.L., Gur R.E. et al. Neuropsychological deficits in neuroleptic naive patients with first-episode schizophrenia. Arch. Gen. Psychiatry, 1994, vol. 51, p.p.124-131] precede the development of manifest psychosis intensifying in exacerbation period and coming back to the "baseline" level at its end [Spaulding W.D., Fleming S.K., Reed D. et al. Cognitive functioning in schizophrenia; implications for psychiatric rehabilitation. Schizophr. Bull. 1999, vol. 25, p.p.275-289]. In this case disorders defined by the term "a neurocognitive deficit" correlate with intensity of negative symptomatology [Harvey P.D., Keefe S.E. Cognition and new antipsychotic. J.Adv. Schizophr. Brain Res., 1998, vol. 1, p.2-8].

Neurocognitive deficiency (poor associative process, disordered attention, memory and productive thinking, lower rate of information processing and performance of executive functions) is considered as a precursor of both disease outcome and patient adaptability [Gold J.M., Harvey P.D. Cognitive deficits in schizophrenia. Psychiatr. Clin. Nortn. Am., 1993, vol. 16, p.p. 295-312; Gallhofer B. Preserving cognitive function - optimizing integration into the community. Schizophr. Rew. 1995, vol.3, Suppl. 2, No.8, p.p.3-4; Stratta P., Daneluzzo E., Bustini M. et al. Schizophrenic deficit in the processing of context. Arch. Gen. Psychiat. 1998, vol. 55, p.p. 186-187; Weickert T.W., Goldberg T.E. The course of cognitive impairment in patients with schizophrenia. Cognition in Schizophrenia. Impairments, Importance and Treatment Strategies. Sharma T., Harvey Ph. (eds.), Oxford University Press, Oxford - New-York, 2000, p.p. 3-16; Breier A.F. Cognitive deficit in schizophrenia and its neurochemical basis. Br. J. Psychiat. 1999, vol. 174, Suppl. 37, p.p. 16-18]. According to a number of investigators [Green M.F. What are the functional consequences of neurocognitive deficits in schizophrenia? Am.J. Psychiat. 1996, Vol. 153, p.p. 321-330; Goldberg T.E., Weinberger D.R. The effects of clozapine on neurocognition; an overview. J. Clin. Psychiatry, 1994, vol. 55, Suppl. B, p.p. 88-90; Harvey P.D., Keefe S.E. Cognition and new antipsychotics. J. Adv. Schizophr. Brain Res., 1998, vol. 1, p.p. 2-8; Sharma T., Harvey Ph. Cognition in Schizophrenia. Impairments and Treatment Strategies. Oxford University Press, Oxford-New-York, 2000, p.p. 363; Mueser K.T. Cognitive functioning social adjustment and long-term outcome in schizophrenia. Cognition in Schizophrenia. Impairments and Treatment Strategies. Sharma T., Harvey Ph. (eds.), Oxford University Press, Oxford-New-York, 2000, p.p. 157-177], neurocognitive deficit phenomena persist in case of complete reduction of clinical symptoms in remission and are even more informative for prognosis of functioning of patients as they may hamper their reintegration into the community.

Therapeutic strategies targeted towards optimization of cognitive functioning of patients may help maintain stable remission, increase their level of functioning and the quality of their life. However, as shown by a series of investigations, remissions with prevalent negative symptomatology and cognitive disorders (including hypochondriac) are characterized by high resistance to medications, including even new-generation drugs [Mosolov S.N. Resistance to psychopharmacotherapy and techniques for overcoming it. Psychiatry and psychopharmacotherapy, 2002, #4, 132-136; Smulevich A.B. Non-manifest stages of schizophrenia - psychopathology and therapy. Zhurn. Neuropat. I Psychiatr. Im S.S.Korsakova, 2005, #5].

In infantile cerebral palsy (CP), clinical manifestations are characterized also by stability and resistance to traditional treatment methods. In pathogenesis of CP clinical signs and symptoms, structural cerebral damage due to the central nervous system (the CNS) disorder and dyscirculatory disturbances secures a leading position. Stability of intellectual-mnestic, emotional-volitional, motor, sensitivity disorders and of other focal signs of brain damage is determined, in particular, by low activity of reparative processes of the CNS in such patients.

The term "cerebral palsy" (CP) comprises syndromes emerging as a result of brain damage at early stages of ontogenesis and manifesting as incapacity to retain a normal posture and perform voluntary movements. Motor disturbances (palsies, pareses, coordination disorders, forced movements) may be associated with changes in mentality, speech, vision, hearing, with spastic fits, and sensitivity disorders. In child development, especially at an early age, clinical symptomatology can change. It is connected with age dynamics of morphofunctional interrelations between pathologically developing brain and increased decompensation determined by growing discrepancy between capabilities of the nervous system and demands raised by the environment to the growing organism. Furthermore, in case of overlay of such pathological symptoms as hydrocephalus, seizures, vegetative disorders as well as tinfectious diseases, intoxications, repeated traumas of the brain, the process progresses.

According to estimates made by a variety of authors in different years and different countries, the incidence of cerebral palsy ranges from 1.5 to 3.0 per 1000 of newborns.

At present, there is no specific therapy enabling to restore dystrophically changed brain tissue. The current treatment methods, including neurosurgical interventions, bear an exceptionally palliative and symptomatic nature.

Thus, the control of CP or hydrocephalus in patients with a resistant neurological deficit has become an exigency determined by a high prevalence of these pathological states, severity of their clinical manifestations and low efficiency of traditional methods of treatment. An urgent need to solve this problem is determined also by a great number of children in whom current treatment methods are ineffective and result in low familial and social adaptation of such patients. The use of umbilical cord blood cells could contribute to acceleration of reparative processes in the CNS by replacement, regeneration or intensification of biological activity of damaged cells resulting in restored or improved function of the brain.

The use of umbilical cord blood cells, which are supposed to have an ability to induce stem cells persisting in the brain to differentiation and promote the normalization of neurogenesis in the brain owing to paracrine effects (the effect of various cytokines and transcription factors), may become an innovation strategy of therapy for diseases proceeding with degenerative changes in the brain.

### Summary of the Invention

This invention concerns using human umbilical cord blood nucleated cells for treatment of mental and neurological disorders bearing no risk of development of immunopathological states, not requiring HLA-typing and without limitations in repeated use in case of clinical indications. Specifically, the invention provides umbilical cord blood cells for use in a method of treatment of a human patient who is suffering from degenerative changes in the brain selected from posttraumatic encephalopathy and cerebral palsy. The invention also provides the use of umbilical cord blood cells for the manufacture of a medicament for use in a method of treatment of a human patient who is suffering from degenerative changes in the brain selected from posttraumatic encephalopathy and cerebral palsy.

In accordance with the invention umbilical cord blood nucleated cells are introduced to the patient suffering from mental or neurological disorders in the therapeutically efficient amount which depending on the patient's weight and clinical presentation ranges from 1x10⁸ to 5x10⁸ and preferably from about 2,2x10⁸ to about 2,8x10⁸ of cells per infusion. Thus, the efficient quantity of nucleated cells in accordance with the invention, at least, is order less than the required quantity of human umbilical cord blood nucleated cells according to the treatment of lateral amyotrophic sclerosis and multiple sclerosis described in the publication WO 2004/071283.

Moreover, unexpectedly to the authors of this invention, they have discovered that the umbilical cord blood nucleated cells are highly effective if applied as a single injection of umbilical cord blood cells to patients. A specific variant of the method application comprises repeated administration of the preparation; in a specific case the preparation is injected up to 4 times to reach a stable remission in schizophrenia patients. Thus, the efficiency of the claimed method significantly surpasses, for instance, the efficiency of the treatment of cerebral ischemia due to atherosclerosis or acute disorder of cerebral circulation described in Patent RU 1 184 190 providing for up to 10 repeated administrations of umbilical cord blood mononuclear cells to patients.

### Detailed Description of the Invention

This invention is based on an unexpected discovery that umbilical cord blood nucleated cells may be injected to patients with mental and neurological disorders without risk of immune rejection resulting in significant amelioration of degenerative diseases and symptoms of consequences of traumatic brain injuries. The invention is specifically concerned with the treatment of cerebal palsy and post-traumatic encephalopathy.

In accordance with the invention umbilical cord blood nucleated cells are used for treatment of mental and neurological disorders in therapeutically efficient quantity by way of intravenous infusion. Depending on the patient's weight and clinical presentation, the amount of infused cells ranges from 1x10⁸ to 5x10⁸ and preferably from about 2,2x10⁸ to about 2,8x10⁸ of nucleated cells per infusion.

In one of variants of the invention the patient is given a single injection of the preparation.

A specific variant of carrying out the invention includes the second infusion of umbilical cord blood nucleated cells depending on clinical presentation, with a 1-4-week interval in the quantity from 1x10⁸ to 5x10⁸ and preferably from about 2,2x10⁸ to about 2,8x10⁸ of nucleated cells per infusion.

In another variant of carrying out the invention, the infusion of umbilical cord blood nucleated cells is administered up to four times with a 1-4-week interval in the quantity from 1x10⁸ to 5x10⁸ and preferably from about 2,2x10⁸ to about 2,8x10⁸ of nucleated cells per infusion.

To obtain the umbilical cord blood cells preparation to be used according to the method, the samples of umbilical cord blood cells are subjected to sedimentation with nucleated cells being selected for further resuspension in cooled plasma with added dimethyl sulfoxide. The material obtained is dispensed into cryo-vials and subjected to program freezing after which it is transferred into a Dewar quarantine vessel with liquid nitrogen vapors. Upon the expiry of the quarantine period of storage in case of negative results of tests for hemotransmissive viral infections and incubation in aerobic/anaerobic conditions, the samples are transferred for permanent storage into the Dewar vessel with liquid nitrogen.

The process used for obtaining the umbilical cord blood cells is permanently reproducible and highly effective enabling to ensure high viability of cells both immediately after harvesting, and after freezing and further thawing.

The preparation presents a sterile suspension of washed-off from cryoprotector viable human umbilical cord blood nucleated cells in the quantity at least 1x10⁸ in 100 ml of physiological saline solution with added rheopolyglukin and human serum albumin, and is designed for intravenous infusion via the blood transfusion system and blood substitute solutions (a dropper), as well as for jet-injection.

In one specific variant of carrying out the invention, umbilical cord blood nucleated cells washed from cryoprotector are used.

In another specific variant of carrying out the invention, umbilical cord blood nucleated cells unwashed from cryoprotector are used.

Primarily, posttraumatic encephalopathy is the consequence of moderate to severe traumatic brain injuries.

The prognosis following mild traumatic brain injury, as a rule, is favorable with nothing but regimen requirements and therapeutic recommendations being observed. Practically, complete convalescence is observed 2-3 weeks after injury, and thereafter such patients do not require follow-up and treatment. In case of cerebral concussion resulting in mild traumatic brain injury, magneto-resonance tomography (MRT) does not reveal any parenchymal focal pathology. Computer tomography (CT) in patients does not identify traumatic deviations in the state of the cerebral matter and liquor-containing intracranial spaces, but a lower-pressure area in the cerebral matter corresponding to brain edema may be revealed (Kornienko V.N., Vasin N.Ya. and Kuzmenko V.A. Computer tomography in diagnostics of craniocerebral injury. M. "Meditsyna", 1987; Kornienko V.N., Lichterman L.B., Kuzmenko V.A., Turkin A.M. Computer tomography. In the book "Craniocerebral Injury. Clinical Guidance", Moscow. "Antidor", 1998, V.1. pp. 472-495). Edema can be local, lobar or hemispheral, and manifests as a moderate voluminous effect in the form of constricted liquor spaces. These changes revealed within the first hours after injury usually reach their maximum on day 3 and disappear in 2 weeks. Thus, morphological changes in this case bear a reversible character, and morphological restoration precedes complete clinical convalescence.

In patients that suffered moderate traumatic brain injury not infrequently even long time after, the signs of posttraumatic psychoorganic syndrome, asthenization, headaches, vegetovascular dysfunction, static or coordination disturbances and other neurological symptoms remain (Actual issues of neurotraumatology, edited by A.N.Konovalov, M). These disorders significantly deteriorate the quality of life, hamper social and professional adaptation and force the patients to make repeated visits to doctors. But, as a rule, medical recommendations are limited by recuperative therapy that gives only a temporary effect.

In case of brain contusion resulting in moderate traumatic brain injury, there is always an area of anatomical destruction of the brain (a contusion area). An impairment of brain tissue integrity can spread over the cortex alone and over the cortex and subcortical structures, and can be accompanied by varied in intensity hemorrhages into crushed areas of the brain tissue (Zotov Yu.V., Schedrenok V.V. Surgery of traumatic intracranial hematomas and foci of brain crushes. L. 1984; Zotov Yu.V., Kasumov R.D., Ismail Taufik. Foci of brain crushes, S.-Petersburg, 1996, P.252). In case of moderate brain contusion in a majority of follow-up cases computer tomography demonstrates focal changes in the form of tiny hemorrhages in the contusion area, moderate hemorrhagic penetration into the brain tissue without its severe destruction. The affected zone is replaced by a glial or gliomesodermal scar 3-4 months later (Lichterman L.B., Fraerman A.P., Khitrin L.Kh., Staging of clinical course of craniocerebral injury, Gorky, 1979, pp. 168-178).

Also disclosed herein is the use of umbilical cord blood cells for treatment of brain damages due to moderate brain contusions. The clinical presentation of an acute period of moderate brain contusion (within first 4-5 weeks) is characterized by the blackout after getting trauma for not more than 2 hours. Transitory disorders of vital functions may occur, namely, bradycardia or tachycardia, BP increase, tachypnea, subfebrility. Tunicary and stem symptoms are often revealed. A focal symptomatology is distinctly manifested, which nature is conditioned by the localization of brain contusion; papillary and oculomotoric disorders, extremity pareses, disorders of sensitivity, speech, etc. Mental disorders may include affective states such as emotional incontinence, tearfulness, dysphoria, asthenic syndrome, memory disorder, including retrograde, anterograde and congrade amnesia, attention deficit (Lichterman L.B., Fraerman A.P., Khitrin L.Kh. Staging of clinical course of craniocerebral injury, Gorky, 1979, pp. 168-178). The following dynamics of the regression of pathological symptoms is observed: within 3-4 days after injury whole-brain phenomena grow. Within this period the state of patients is of moderate severity. It improves in 2 weeks, whole-brain and meningeal symptoms ameliorate. Vegetative disorders remain manifest; subjective and objective signs are without substantial changes. By the forth week, subjective signs include only: moderate headache, vertigo, tinnitus, ambliopia, phenomena of asthenia and vegetovascular instability (Lichterman L.B., Fraerman A.P., Khitrin L.Kh. Staging of clinical course of craniocerebral injury, Gorky, 1979, pp. 168-178).

A subacute period of brain contusion (up to 4 months) is characterized by cerebroasthenic disorders with irritability, sensitivity, vulnerability, tearfulness, accelerated cachexia and lowered performance efficiency. The patients complain of sleep disorders, heat and stuffiness intolerance, a sense of faintness when riding in a transport vehicle and insignificantly lowered memory. Hysteroid reactions, moderate affective disorders may appear. The objective examination reveals insignificant disseminated neurological symptomatology, vaso-vegetative disorders (Lichterman L.B., Fraerman A.P., Khitrin L.Kh. Staging of clinical course of craniocerebral injury, Gorky, 1979, pp. 168-178). The long-term consequences of moderate traumatic brain injury can manifest up to 2 years and over.

In another variant, the claimed invention is directed to using umbilical cord blood cells for treatment of encephalopathy due to severe traumatic brain injury. In case of severe traumatic brain injury that can be caused by heavy brain contusions, diffuse axonal damage and cerebral compression, the prognosis often is unfavorable with lethality rate reaching 15-30%. The survived suffer from significant disability, which leading causes include mental diseases, epileptic seizures, severe motor and speech disorders (Manevich V.N., Manevich A.Z., Potapov A.A., Bragina N.N. Pathophysiological basics of intensive therapy for severe craniocerebral injury, Vest. AMN USSR, No.6, p.60, 1986). Such disorders require long-term complex and specific treatment. Even in case of a favorable course of the posttraumatic process, the convalescence period usually protracts for many months, and sometimes years. Computer tomography of severe contusions reveals fresh blood clots, edematous and/or crushed brain tissue with zones of atrophy or cystic cavities formed at the site of damage (Kornienko V.N., Vasin N.Ya. and Kuzmenko V.A. Computer tomography in diagnostics of craniocerebral injury. M. "Meditsyna", 1987).

During application of the claimed invention, administration of umbilical cord blood stem cells concentrate was unaccompanied by any reactions on the part of cardiovascular, respiratory or immune systems.

In variants of application of the claimed method targeted to the treatment of posttraumatic encephalopathy due to moderate-to-severe traumatic brain injuries, after administration of cells all patients demonstrated a distinct tendency towards rapid (within three months) reduction of all structural components of asthenic syndrome: increased fatigability, cachexia, affective lability, hyperesthesia, phenomena of vegetative dysfunction, drowsiness. The level of mental activity was raised considerably. Three months after administration of umbilical cord blood cells, the patients with paresis demonstrated its practically complete regression. The patients with aphasic disorders demonstrated substantial amelioration of speech functions: elements of motor aphasia regressed, speech rate increased.

Thus, the patients with various neurological symptoms suffering from consequences of severe-to-moderate traumatic brain injuries showed observable amelioration of their state. The findings obtained suggest that therapy using umbilical cord blood cells for posttraumatic encephalopathy in accordance with the claimed invention unexpectedly produces rapid antiasthenic and nootropic effects accompanied by accelerated restoration of cognitive and "instrumental" cortical functions.

Also disclosed herein is the use of umbilical cord blood cells for treatment of patients with Parkinson's disease having a stable neurological deficit at the background of anti-parkinson therapy administered.

Neurological deficiency in patients was assessed according to standard for this disease tests with the use of Columbian Rating Scale (CRS), Yahr M.D. et al. Treatment of parkinsonism with levodopa. Archives of Neurology, 1967, v. 21, P.343-354; Lang A.E.T., Fagn S. Assessment of Parkinson's disease In: Quantification of neurological deficit (ed. T.L., Munsat). Butterworth, Stoneham, 1989, Ch.21. P.285-309; Wade D.T. Measurement in neurological rehabilitation. Oxford University Press. 2000) and Parkinson's Disease specific measure of life quality (PDQ-39 Scoring System, Loma Linda University Surgery Medical Group).

The study enrolled patients aged from 48 to 86 years with Parkinson's disease stage 2 and 3, moderate type of progression, rigid-trembling and akinetic-trembling forms, disease duration from 3 to 10 years.

No adverse effects have been revealed in all patients enrolled in the study either within the treatment process, or three months afterwards.

As a result of treatment using the claimed invention, all patients have demonstrated increased functional activity, i.e., an expanded scope of everyday activity at home, an increased motivation degree and an improved emotional component of their state. A positive effect was developing during the first month and was characterized by improved state of the skin in terms of decreased greasiness, seborrhea and manifestations of clinical phenomena of the disease.

As a result of testing, statistically significant differences have been revealed as well with regard to rigidity, bradykinesia and functional abilities by the end of third month from the start of treatment. By the end of the first month of the study, the patients with trembling forms have demonstrated distinctly a decrease in tremor. Rigidity has undergone an evident regression with a gradual decline of manifestations by third month. Bradykinesia, as the most disabling sign, has demonstrated more distinct regression in patients with a prevailing akinetic component. The expanded range of functional abilities has been distinctly registered in all patients already by the end of the first month.

Thus, based on data obtained in testing by PDQ-39 Scoring System, positive dynamics has been observed in all patients by the end of third month.

It should be noted that an absolute positive result of this method of treatment is the lowered dose of replacement therapy with L-DOPA drugs in the follow-up process.

The use of the umbilical cord blood nucleated cells in Parkinson's disease is well tolerated by patients and does not show any negative side effects. According to assessment of clinical data, the use of this method has a positive effect upon the course of the pathological process in terms of a moderate regression in main symptoms of disease. The dose reduction of L-DOPA drugs at the background of patients' stable state is also an absolute important technical result achieved with the use of this method.

The simplicity of application combined with the efficacy guarantee the advantages of the above as a new and perspective direction in treatment of Parkinson's disease.

Also disclosed herein is a treatment of Alzheimer's disease. The study enrolled patients aged 50-85 years with moderate severity dementia by the CDR criteria [Morris J.C. The Clinical dementia rating (CDR). Current version and scoring rules, Neurology, 1993, Vol. 43, p. 2412-2413].

After preliminary somatic, neurological and laboratory examinations (clinical blood count and urinalysis, ECG), all patients received umbilical cord blood nucleated cells by i.v. drop infusion in the quantity from 1x10⁸ to 5x10⁸ and preferably from about 2,2x10⁸ to about 2,8x10⁸ of nucleated cells per infusion with a 1-week interval.

The state of cognitive abilities (MMSE Scale, Mini-Mental-State Examination, Folstein M.F., Folstein S.E., McHugh P.R. Mini-Mental State. A practical method of grading the cognitive state of patients for the clinician. Journal of Psychiatry Research, 1975, Vol. 12, p. 189-198), disorders of cognitive functions (ADAS-cog Scale), disorders in everyday activities (Bristol-ADL Scale), quantitative index of the state of higher cortical functions (according to the A.R.Luria adapted technique) and clinical general impression (GGI Scale) were estimated 7, 14 and 90 days after the commencement of therapy by the method. Simultaneously, the main indications of the organism state (pulse rate, clinical blood count, urinalysis and ECG) were assessed prior to and after the completion of the study.

Within the study period, 4 in 5 patients continued to take the drugs traditionally used for treatment of Alzheimer's disease, namely, one of acetyl cholinesterase inhibitors or memantin in stable doses.

Primary criteria for efficiency evaluation of using the umbilical cord blood nucleated cells according to the invention were dynamics of indicators of cognitive functions disorders by ADAS-cog Scale and a change in indicators of everyday functioning disorders by Bristol-ADL Scale. Secondary criteria for efficiency evaluation were dynamics of indicators of cognitive functioning by MMSE Scale and assessment of clinical general impression (GGI Scale).

Positive dynamics in the state of cognitive functions by scales ADAS-cog and MMSE was observed in all patients on days 7 and 14 of the study, i.e., a week after the first and second injection of the umbilical cord blood nucleated cells.

Positive dynamics was also observed in everyday functioning of patients (by Bristol ADL scale) on day 14 of the study. i.e., a week after the second injection of the umbilical cord blood cells preparation. On day 90 of the study 3 in 5 patients preserved positive dynamics and stable state achieved by day 14 of the study by ADAS-cog, Bristol-ADL and MMSE scales.

According to the scale of general clinical general impression (CGI) on day 14 of the study, 2 in 5 patients demonstrated observable improvements in cognitive and everyday functioning accompanied by improved memory, orientation and speech, as well as by restoration of lost habits of personal hygiene and expansion in the scope of everyday activities, for instance, in house cleaning, preparation of drinks.

Ninety days later, the state of 2 patients was assessed as moderate or minimum improvement compared to the prior-therapy estimates.

The advantages of the above manifesting in positive prolonged dynamics of cognitive and everyday functioning without side effects demonstrate a perspective of its use in treatment of Alzheimer disease.

Also disclosed herein is the use of umbilical cord blood stem cells for treatment of patients with primary-progradient and secondary-progradient type of multiple sclerosis. The patients received twofold injection of blood-group and rhesus factor compatible umbilical cord blood cells in the amount of 1x10⁸ to 5x10⁸ and preferably from about 2,2x10⁸ to about 2,8x10⁸ of nucleated cells per infusion with a 14-day interval. After repeated administration within 1-month follow-up, positive dynamics was observed in all patients.

Also disclosed herein is the use of umbilical cord blood stem cells for treatment of schizophrenia patients.

The structure of remission in schizophrenia is characterized by prevailing negative symptomatology (emotional insulation, difficulties in communication, loss of energy and volitional incentive) in combination with pronounced cognitive impairment (slipping, sperrung, diverse thinking) and subdepressive disorders with negative affectation (apathy, a sense of personal inefficiency) [Smulevich A.B. Non-manifest stages of schizophrenia - psychopathology and therapy. Zhurn. Neuropat. I Psychiatr. Im S.S.Korsakova, 2005, No 5].

Treatment was administered in 10 patients, aged on the average 33.6 ± 10.6 years, in a state of hypochondriac remission with cognitive deficits prevailing in the structure of the defect, with average duration of disease 17.3±4.1 years, average number of relapses 44.4±2.2 and average duration of remission 36.7±24.8 months. The control group (10 patients with the same diagnosis comparable by gender, age, disease parameters) received placebo.

Umbilical cord blood cells were introduced by way of i.v. drop infusion in the quantity of 1x10⁸ to 5x10⁸ and preferably from about 2,2x10⁸ to about 2,8x10⁸ of nucleated cells per infusion in 4 injections with a 14-day interval at the background of supporting therapy (stable doses of antipsychotics).

The patients' state was assessed clinically, psychometrically and pathopsychologically before each injection, and 3, 5 and 7 weeks after the last procedure and 3 months later in a pilot phase, as well as 9, 11 and 13 weeks later in a clinical phase.

Pathopsychological assessment was held with the use of known test methodology - the MCCB cognitive battery (The MATRICS Consensus Cognitive Battery) [Nuechterlein K.N., Green M.F., Kern R.S., Baade L.E., Barch D.M., Cohen J.D. et al. The MATRICS Consensus Cognitive Battery, Part 1: Test Selection, Reliability, and Viability. Am. J. Psychiatry, 2008, Vol. 165, p.p. 203-213].

The battery includes 10 tests assessing 7 cognitive spheres and targeted to estimate the following parameters: conscientious control of emotions for personal growth and improvement of interpersonal relations (social intellect), visual-motor coordination (attention, special orientation, motor coordination, the speed of intellectual processes); verbal memory and verbal learning; verbal associative productivity and lexical system disorder; executive functions (planning, programming, modeling and control of mental activity); ability to symbol coding (attention, work memory and the speed of intellectual processes); visual memory and visual learning; attention (scope, concentration, switching and awareness); nonverbal (spatial) work memory; the ability to implement analytico-synthetic operations and associative process.

The Positive and Negative Syndrome Scale (PANSS) was used for psychometric assessment of positive and negative symptomatology of schizophrenia [Kay S.R., Fiszbein A., Opler L.A. The Positive and Negative Syndrome Scale (PANSS) for Schizophrenia. Schizophr. Bull., 1987, Vol.12, p.p. 261-276; Mosolov S.N. Scales for psychometric assessment of schizophrenia symptomatology and the concept of positive and negative disorders. M., 2001, p.238].

No side effects were observed in any case of administration of the umbilical cord blood nucleated cells.

Transitory episodes of low fever (t° up to 37.1-37.2°C) did not require additional interventions and were arrested aidless within 1-2 days.

An improvement in the patients' state was recorded at the pathopsycological examination (the MCCB indicators) as soon as 3 weeks after a single infusion of the preparation of umbilical cord blood nucleated cells with baseline values coming back 5-7 weeks later.

A series of 4 consecutive infusions of the preparation renders a positive effect on cognitive functioning.

A significant improvement is recorded at the pathopsycological level with a high statistical significance (p<0.001). In three weeks after the fourth infusion, the speed of mental processes increases significantly (dynamic characteristics of attention, visual-motor coordination, associative productivity of thinking, regulatory functions, the rate of skills formation). Attention/awareness (scope, concentration, switching, distribution) and memory improve. The ability to learn, including visual (learning the material in visual modality) and verbal (learning the material in audio-speech modality) abilities, increases. The capabilities associated with executive functions (processes of planning, programming, modeling and control of mental activity are optimized) expand.

The effect reached in accordance with the claimed invention distinguishes itself not only by its stability, but also by duration (positive dynamics of parameters of cognitive functioning is recorded 3 and 5 weeks after the last infusion, and starting from 7 to 13 weeks is preserved at a plateau level).

Statistically significant (p<0.05) improvement in psychometric indicators assessed by the PANSS scale is recorded for "disorder in speech spontaneity and smoothness", (the initial total score equaled to 3.0±0.71), and after a series of 4 infusions it fell to 2.3±0.27; for "depression" the total score fell to 2.2±0.45 from the initial values of 3.0±0.71; for "motor inhibition" after treatment the total score fell to 2.3±0.45 from the initial values of 3.0±0.71.

The psychometric data obtained are in agreement with clinical findings (an improved state of patients is limited by the development of optimistic mood, revived mimics and pantomimics, signs of interest in communication, care about their appearance).

The cognitive effects reached after infusions of the umbilical cord blood nucleated cells to patients is a state of hypochondriac remission according to the claimed method result in pronounced metabolic (nootropic) and psychostimulating effects manifested in activated intellectual activity, accelerated processes of thinking, corrected mnestic functions and increased level of wakefulness. The effectiveness of the method with regard to improved cognitive functions distinguishes itself not only by stability, but also by persistence.

The claimed invention may be directed to using umbilical cord blood nucleated cells for treatment of patients with infantile cerebral palsy and hydrocephalus.

Morphologically, cerebral palsies are characterized by structural changes in the central nervous system varied in nature, severity degree and localization. Peculiarities of these disorders depend, mainly, upon time of action of pathogenic factors and, in lesser degree, upon specific properties of the latter. Developmental defects of brain hemispheres in infantile cerebral palsy can be associated either with hydrocephalus due to inborn abnormal development of the spinal fluid circulation or changes in the meningeal structure in subarachnoidal hemorrhages, inflammatory processes, etc. Changes in the brain occur in intrauterine fetal hypoxia and newborn asphyxia of dyscirculatory genesis. Their intensity depends on hypoxia duration and severity. Vascular disorders and edema create conditions for secondary ischemia of the nervous tissue. In the substantia alba of the cerebral hemispheres, especially in subependymal compartments, the foci of intensive edema are formed which later on can transform into the foci of encephalomalacia and porencephalia. In case of prolonged hypoxia, the neurons undergo dystrophic changes in the form of chromatolysis, central acidophilia, acute swelling. Ischemic changes of neurons are accomplished by cellular lysis ("cells-shadows').

Thus, deep circulatory disorders (edema, hemorrhagia, thromboses, ischemia) developing under the effect of unfavorable external impacts in ante- and intranatal periods result in dystrophic changes in the brain tissue lying in the basis of infantile cerebral palsy. In immature infants the substantia alba of the brain is damaged more frequently. Increased vulnerability of the substantia alba in the developing brain results from its high level of metabolism compared with the cortex accompanied by a relatively poor blood supply (C.Kennedy et al. 1979).

Other regions such as subcortical substantia alba, corpus collosum and internal theca can also undergo necrosis.

The umbilical cord blood nucleated cells in accordance with the invention were administered to 19 patients aged from 1 year 3 months to 10 years, 17 of which were disabled since birth. The pathological processes included hydrocephalus (in 2 patients), infantile cerebral palsy (4 patients), hydrocephalus accompanied by CP and epileptic syndrome (4 patients), hydrocephalus with epileptic syndrome (7 patients), hydrocephalus with CP (2 patients). All patients received treatment in various specialized clinics, including from 3 to 8 inpatient hospitals without any positive effect. In all cases the pathological process at the background of treatment had a progradient course.

Major clinical manifestations of disease were spastic hemiparesis (in 8 patients), tetraparesis (4 patients) epileptic syndrome (11 patients); retarded psychomotor and physical development was observed in all 19 patients.

Taking into consideration the severity of clinical presentation of disease and the failure of medication therapy, single (8 patients), twofold (10 patients) and threefold (1 patient) transfusions of blood group and rhesus factor compatible umbilical cord blood cells in the total dose from about 100 to about 600 mln cells were performed.

Using the claimed invention, all patients have demonstrated positive dynamics. Decreased spastics and hyperkinesis were observed in 5 patients. One female patient showed regressed right-sided hemiparesis on day 2 after the second infusion of cells (from 2 to 4 points), the rate of clonic twitchings in her arm decreased. Another patient with inborn amaurosis demonstrated improved visual functions: the boy started to describe the surrounding objects and to name the objects depicted in pictures; improved minor motor activity and first skills of self-servicing were also observed. Patients with infantile cerebral palsy or hydrocephalus associated with epileptic syndrome have demonstrated a lower rate of seizures without any change in the dose of anticonvulsant drugs. The stabilized state was observed in 9 patients.

It should be noted that in carrying out all specific variants of the claimed invention, the infusion of the umbilical cord blood nucleated cells was not accompanied by any reactions on the part of cardiovascular, respiratory or immune systems.

In the variants of carrying out the claimed invention, after infusion of cells all patients suffering from mental or neurological disorders, have demonstrated a distinct tendency to rapid reduction of structural components of degenerative disorders (symptomatology), the level of mental activity essentially increased.

Accordingly, the state of patients with different neurological symptomatology, suffering from degenerative pathological disorders has been clearly ameliorated. The findings obtained suggest that using umbilical cord blood cells in accordance with the claimed invention for treatment of mental and neurological disorders unexpectedly results in rapid nootropic and psychostimulating effects accompanied by accelerated restoration of cognitive and "instrumental" cortical functions.

The following examples illustrate, but not limit, the claimed scope of invention.

### Example 1. Collection of umbilical cord blood and isolation of nucleated cells.

Umbilical cord blood was collected into sterile bags (the systems for collection of donor blood) containing an isotonic anticoagulant with precautions taken against contamination of the substance during its collection and transportation. The substance was delivered to the laboratory in an insulated shipping container at room temperature (+18... +22°C).

All isolation procedures of umbilical cord blood nucleated cells were carried out in accordance with the national standard of the Russian Federation GOST 52249-2004 "The regulations for production and quality control of medications" with the use of medical processing technique developed by the authors of the invention.

After the volume of umbilical cord blood is measured, aliquots are selected to conduct hematological analysis (the level of blood corpuscles before isolation, blood group, rhesus- factor).

To obtain nucleated cells by sedimentation, 6% hydroxyethyl starch solution (HES) is added to the container with blood calculated as 1 volume of HES to 4 volumes of blood, and is agitated for 10-15 minutes. The contents of containers are transferred into 50 ml test-tubes, centrifuged for 5 min at 90 g with braking switched off and allowed to stand for 10-15 minutes for observable separation between leukocyte-enriched plasma and erythrocyte pellet is formed. Leukocyte-enriched plasma is transferred into separate test-tubes and centrifuged for 10 min at 600g to settle down blood cells. The obtained cellular pellets are combined and resuspended in plasma. The remains of plasma are transferred into a separate test-tube to be placed into a container with ice.

When the automatic separator of cells "SEPAX" is used, after the calculated quantity of HES is added, the bag with blood is connected to the separation kit via a sterile port, and the separation is carried out in accordance with instructions for use of the device.

To obtain nucleated cells using the gradient centrifugation method, blood is distributed into 50 ml test-tubes and is centrifuged for 10 minutes at 600g to settle down blood cells. A sample of obtained plasma is transferred into a clean test-tube and placed on ice. Hank's or Earl's phosphate-saline solution is added to cellular pellets and resuspended. Into each 50 ml sterile test-tubes, 20 ml of Ficoll solution is added. Cell suspensions are layered onto the Ficoll solution and centrifuged for 30 min at 400-500g. The interphase mononuclear ring is collected and transferred into clean test-tubes. The phosphate-saline solution is added up to 50 ml into each test-tube, resuspended and centrifuged for 10 min at 600g. The washing-off procedure is conducted twice. Cellular pellets are consecutively resuspended in cooled plasma.

The aliquots of cell suspensions are used to carry out tests for sterility, to identify causative agents of hemotransmissive viral infections, to calculate the number of cells and their viability using the Trypan blue test, to determine the amount of CD34/CD45-positive cells by the flow cytometry.

To the remaining volume of plasma, dimethyl sulfoxide (DMSO) is added to make the final concentration of 20%. The volume of plasma equal to DMSO is added to the cell suspension drop-by-drop under constant stirring. The obtained material is distributed into cryovials, subjected to program freezing, and afterwards is transferred into a Dewar quarantine vessel with nitrogen vapors. Upon the expiry of the quarantine period for storage, providing negative test results are obtained for hemotransmissive viral infections and incubation in aerobic/anaerobic conditions, the samples are transferred for permanent storage into the Dewar vessel with liquid nitrogen.

The samples of umbilical cord/placental blood are tested to identify causative agents of hemotransmissive viral infections: the human immunodeficiency virus (HIV-1/2, antigen/antibody), hepatitis B viruses (HBs AG, anti-HBc-total) and hepatitis C virus (anti-HCV-total), T-cell lymphotropic virus (anti-HTLV-1/2), type 1 and 2 herpes simplex virus (anti-HSV IgM), cytomegalovirus (anti-CMV IgM), toxoplasmosis causative agents (anti-Toxo IgM), syphilis (Syphilis PRP), bacterial and fungal agents.

### Example 2. Preparation of the dosage form of cryopreserved umbilical cord blood cells preparation.

The preparation presents a sterile suspension of washed from cryoprotector viable nucleated cells of human umbilical cord blood in the quantity at least 1x10⁸ in 50-100 ml of saline solution with added rheopolyglukin and human serum albumin, and is designed for intravenous infusion via the blood transfusion system and blood substitute solutions (a dropper), as well as for jet infusion.

While the cell suspension is thawed, a needed quantity of cryovials with frozen cells is extracted from the cryogenic storage to stand for 5 min at room temperature.

With protective packing left in place, cryovials are placed into the water bath (+37oC) and thawed till disappearance of ice crystals. By overturning test-tubes, the contents are mixed regularly. Cryovials are disclosed by unscrewing the cap with internal threading.

Using 1 ml pipette dispensing apparatus or a tipped dispenser, the contents of each cryovials are transferred into 50 ml test-tubes.

Cooled washing solution is added drop by drop while stirring (first 10 ml), then jet-like to make a final volume 45 ml. The contents are resuspended by repeated pipetting and the test-tubes are closed by caps.

The contents are centrifuged for 10 min at 600 g and the supernatant is removed using a pipette dosimeter or an aspiration pipette and a medical suction device. About 1 ml of liquid is left at the bottom of each test-tube.

Cellular depositors are consequently resuspended into 10 ml of cooled solution for washing off, avoiding foaming production. Using a microdosimeter, 100-200 microliters of cell suspension are transferred into a micro test-tube to assess viability and calculate the number of cells. Using a 20 ml or 50 ml syringe equipped with a needle, the remainder cell suspension is transferred into a vial or a polymer container. Using a 50 ml syringe, the saline solution with rheopolyglukin and albumin is added to the vial (a polymer container) to make the final volume of 100 ml. The content and viability of cells in suspension is determined using the material from micro test-tube.

The time between preparation of the dosage form (the transfer of washed-off cells into a vial or a polymer container) and the commencement of infusion should not exceed 4 (four) hours. Vials (polymer containers) are transported to the place of infusion administration at a temperature of +2-4oC (on ice) in insulated shipping containers.

Reference Example 3. Treatment of Alzheimer's disease.

A female patient, 68 years old, has been followed up from 65 years old for Alzheimer's disease. Initial memory disorders appeared at the age of 60 years. The MRT investigation of the head has revealed enlargement of lateral ventricles and subarachnoidal spaces in the projection of frontal, parietal and temporal lobes with the diagnosis of external and internal communicating hydrocephaly. Her orientation in time and place is disordered. Short-term and long-term memory disorders are manifest, she fails to date events.

Memorizing a series of simple words is difficult. Her speech is characterized by paraphrasias and single logoclonias. She fails to tell the time by the clock, cannot memorize any human face. Real objects can be recognized with errors. The patient confuses the succession of postures when performing tests for investigation of dynamic praxis. Spatial-constructive activity is disordered in a severe degree (at redrawing of three-dimensional geometric figures she makes significant mistakes). Intellectual functions per se are disordered moderately: the patient is confused in interpretation of logical-grammatical structures, understands directly the figurative meaning of many proverbs, misunderstands subject images. The computation function is disordered: makes mistakes at consecutive subtraction, in examples for addition with two-digit numbers. Writing disorder includes the following: she wrights each letter separately and large size. Reads correctly, memorizes the text fragmentary. The treatment with Exelon (Rivastigmin) by 1.5 mg twice daily has not resulted in amelioration.

At the background of continued Exelon treatment, two drop-by-drop intravenous infusions of washed from cryoprotector, blood-group and rhesus-factor compatible umbilical blood cord cells were administered to the patient in the quantity of 2.5x10⁸ per infusion with a two-week interval. After the first infusion the female patient has become more energetic, better recollected words during conversation, the ability to perform some household routines was restored. At the follow-up on day 7 post-infusion 1, an improvement in memorizing and recognition of words, in her spoken language ability and time orientation was observed.

On day 14 after the commencement of treatment an improvement in place and time orientation, memorizing a series of words and naming objects and fingers of the hand, and further better recognition of words was noted; the search for words in voluntary speech was easier and the number of performed commands increased. She began to prepare drinks correctly and to perform housework. The MMSE and ADAS scores improved by 7 points and 14 points, respectively, compared with the initial assessment of cognitive functioning.

Three months after the commencement of treatment, the patient's state remained at the level achieved by day 14 of the study.

Reference Example 4. Treatment of Parkinson's disease.

A male patient, 64 years old, with akinetico-rigid form stage 2 Parkinson's disease received Madopar by 250 mg in a daily dose of 1250 mg, Mydocalm by 100 mg four times daily, Pronoran by 50 mg thrice daily, PK-Merz by 100 mg thrice daily on a constant basis.

Before treatment, the Columbian Rating Scale score equaled to 32 and PDQ-39 Scoring System score equaled to 30.

Umbilical cord blood cells in a dose of 2.59x10⁸ were transfused to the patient intravenously. On week 4 post-infusion, positive dynamics appeared in terms of decreased muscular constraint and a positive cosmetic effect. The dosage of antiparkinsonian drugs was substantially decreased:
^{m}adopar up to 500 mg daily, Pronoran up to 100 mg daily, PK-Merz up to 300 mg daily. Three months later the score by the Columbian Rating Scale and by Parkinson's Disease specific measure of life quality (PDQ-39 Scoring System) decreased to 16 and 28 points, respectively.

Reference Example 5. Treatment of Parkinson's disease.

A female patient, 58 years old. Weakness and tremor in the left arm, weakness in the left leg and in the right leg, constraint in the whole body, motor deceleration, urinary incontinence. Recommendations for constant administration included Madopar in a dose of 125 mg by 1 capsule four times daily every 6 h. She walked for short distances aidless with her body inclined forward. Propulsions were noted during walks. The score by the Columbian Rating Scale and by Parkinson's Disease specific measure of life quality (PDQ-39 Scoring System) amounted to 44 and 100 points, respectively.

Blood-group and rhesus-factor compatible umbilical blood cord cells in a dose of 2.49x10⁸ were transplanted to the patient by way of intravenous infusion. After infusion, observable positive dynamics included diminished phenomena of seborrhea dermatitis on her face, improved mood, less constraint of the body, higher tolerance to physical loads and less postural instability. The score by the Columbian Rating Scale and Parkinson's Disease specific measure of life quality (PDQ-39 Scoring System) decreased to 18 and 56 points, respectively.

Reference Example 6. Effect upon negative symptoms and cognitive deficit in case of incomplete remission in schizophrenia.

A male patient, 24 years old, after 5-month in-patient treatment for diagnosis of "Schizophrenia paranoid, episodic type with a growing defect, paranoid syndrome" (acute manifest paranoid psychosis with delusion of grandeur, Kandinsky-Clerambault syndrome), when he received Aminasin, Haloperidol, Zyprexa, Rispolept, Cyclodol and experienced fatigability, apathy, low spirits, taking as a supportive therapy Haloperidol (10 mg daily), then Rispolept 96 mg daily), in a state of incomplete remission within the framework of paranoid schizophrenia with a prevailing negative symptoms and manifest cognitive disorders was subjected to treatment.

Prior to treatment, positive symptomatology, negative symptomatology and general psychopathology by PANSS score corresponded to 9, 20 and 40 points, respectively. Tests with the use of the MATRICS cognitive battery demonstrated foreground disorders in such cognitive spheres as attention with T-score of 25 (0.65%), visual learning with T-score of 21 (0.2%), the speed of mental processes with T-score of 34 (5%) and regulatory functions with T-score of 34 (5%); work memory (verbal and visual-dimensional) with T-score of 45 (31%) and verbal learning with T-score of 48 (42%) were also deficitary.

At the background of stabile rispolept therapy in a dose of 6 mg daily, a series of infusions (4 injections) of blood-group and rhesus-factor compatible umbilical blood cord cells was administered in the quantity of about 2.5x10⁸ per infusion (a total dose about 1x10⁹ cells) with a 14-day interval.

Cognitive indicators measured 9 weeks after a series of infusions of the umbilical blood cord cells have demonstrated improved parameters of cognitive functioning with a high statistical confidence: T-score of 51 (54%) for the speed of mental processes, T-score of 63 (90%), for work memory, T-score of 78 (99.7%) and 60 (84%) for verbal and visual learning, respectively; T-score of 59 (82%) for executive (regulatory) functions. The most manifest effect is observed in such cognitive spheres as learning (visual and verbal) consisting in the ability to memorize material in visual and audio-linguistic modalities and executive functions responsible for the processes of regulating, planning, programming and control over mental activity. According to the PANSS assessment 9 weeks after a series of infusions of the umbilical cord blood cells, positive symptomatology, negative symptomatology and general psychopathology scores were 9, 14 and 36, respectively. Higher spirits (reduced oppressiveness), a lower degree of fatigability and apathy, and the appearance of an optimistic mood were observed.

The degree of recovery of executive functions, visual and verbal learning as well as memory and the speed of mental processes within the post-therapeutic period (week 9) reaches the level of overall average norm, i.e., exceeds the average T-score of 50 and 50 percentiles, respectively, according to the MATRICS cognitive battery.

Thus, 9 weeks later a distinct trend to recovery of all said cognitive functions was observed in the patient. The treatment resulting in the optimization of cognitive functioning of patients, maintains stable remission, an increased level of functioning and the quality of their life.

Reference Example 7. Effect upon negative symptoms and cognitive deficit in case of incomplete remission in schizophrenia.

A male patient, 47 years old, suffers from "schub"-like schizophrenia since the age of 31 years with predominant affective-paranoid psychoses (mainly depressive-paranoid episodes with manipulation syndrome, delusion ideas of guilt and self-annihilation), experienced 6 attacks with hospitalizations to mental clinics, 2 suicidal attempts in anamnesis.

Disabled; hypochondriasis, apathy, fatigability predominate in the presentation of remission, every year seasonal depressive phases with a sense of vital anxiety, circadian rhythm, weight loss are observed. Received maintenance therapy: Rispolept 8 mg daily, Carbamazepin 200 mg weekly, Amitriptyllin 50 mg daily.

The patient in the state of incomplete remission within the framework of "schub"-like schizophrenia determined primarily by negative symptomatology, depressive disorders and manifest cognitive disorders underwent treatment using the claimed method. Prior to treatment, positive symptomatology, negative symptomatology and general psychopathology scores according to the PANSS assessment were 12, 24 and 44 points, respectively. Tests with the use of the MATRICS cognitive battery demonstrated foreground disorders in such cognitive spheres as the speed of mental processes with T-score of 32 (3.6%), work memory (verbal and visual-spatial) with T-score of 37 (10%), verbal learning with T-score of 34 (5%) and executive functions with T-score of 36 (8%). The disordered scope and dynamic characteristics of attention with T-score of 41 (18%) were also revealed.

At the background of fixed doses of supporting therapy, a series of infusions (4 injections) of blood-group and rhesus-factor compatible umbilical cord blood cells were administered in the quantity of about 2.5x10⁸ per infusion with a 14-day interval.

Cognitive indicators measured 9 weeks after a series of infusions of the umbilical cord blood cells have demonstrated an improvement in all parameters of cognitive functioning with a high statistical confidence, namely, T-score of up to 69 (97.1 %) for the speed of mental processes, T-score of up to 67 (96%) for attention, T-score of up to 66 (95%) for work memory, T-score of up to 61 (86%) and 72 (98.6%) for verbal and visual learning, respectively; and T-score of up to 64 (92%) for executive (regulatory) functions. The most manifest effect was observed in such cognitive spheres as the speed of mental processes, executive functions, spontaneous memory (verbal and nonverbal) and verbal learning. According to the PANSS assessment 9 weeks after a series of infusions of the umbilical cord blood cells, positive symptomatology, negative symptomatology and general psychopathology scores were 10, 18 and 39, respectively. Oppressiveness, anxiety reduced observably, the patient became more companionable, started to care about his appearance.

The degree of recovery of executive functions, visual and verbal learning as well as memory and the speed of mental processes within the post-therapeutic period (week 9) reaches the level of overall average norm, i.e., exceeds the average T-score of 50 and 50 percentiles, respectively, according to the MATRICS cognitive battery.

Thus, 9 weeks later a distinct trend to recovery of all said cognitive functions was observed in the patient. The treatment method resulting in the optimization of cognitive functioning of patients maintains stable remission, an increased level of functioning and the quality of their life.

Example 8. Cerebral palsy with epileptic seizures.

A 9-year-old female patient with diagnosis: chronic advanced focal Rasmussen's encephalitis, epilepsy with frequent simple partial motor attacks, myoclonic and secondary-generalized seizures, right-sided hemiparesis. Received Keppra by 750 mg twice daily, Pagluferal-3 by ½ tabs twice daily, Trileptal by 150 mg thrice daily. In spite of administered therapy, advanced deterioration of her state was observed with accelerated attacks of unchanged or partially impaired consciousness, abduction of head to the right and tensioning of the right extremities with development of post-attack Todd's paralysis non-arrested by Seduxen.

Epileptiform activity was recorded by encephalography (ECG). Magneto-resonance tomography revealed the picture of changes characteristic of atrophic process phenomena in the left hippocampus, pathologic foci in cortical zones of left temporal and parietal regions.

Considering the duration and the failure of administered conservative therapy, the occurrence of frequent epileptic seizures and retardation in psycholinguistic development, twofold intravenous infusions of the human umbilical cord blood cells in a total dose of about 5x10⁸ cells (2.46x10⁸ and 2.54x10⁸, respectively) were administered to the child with a 1-month interval.

The following day after the second infusion of human umbilical cord blood cells, positive dynamics was observed in the girl: right-sided hemiparesis decreased especially in the upper extremity (up to 4 points), the girl began to lift the right extremity vertically. Twitching in the right arm decreased.

The follow-up examination 5 months later has revealed that at the background of administration of anti-epileptic drugs the seizures have completely disappeared. The degree of hemiparesis decreased up to 3 points. The ECG recorded positive dynamics. The quality of life has significantly improved: her speech improved, the child could care about herself independently and danced a lot.

### Example 9. Cerebral palsy with hydrocephalus.

A male patient, 7 years 5 months, with diagnosis: mixed hydrocephalus, retarded psychomotor and linguistic development, inborn two-sided amaurosis, with a sharp decline of optical functions up to light perception; at the background of administered medication therapy there was no improvement in his state.

The ECG data suggested the presence of disseminated changes with stem formations involved into the process with disarranged cortical-subcortical interrelations.

Considering the severity of the clinical presentation, findings of interoscopy, a progradient course of disease, the failure of medication therapy, three infusions of umbilical cord blood cells in the total dose of about 6x10⁸ cells (about 1.03x10⁸, 2.5x10⁸ and 2.5x10⁸ per infusion, respectively) were administered to the child with a 1-month interval.

Stable positive dynamics was observed in the boy 3 months after the last infusion. He became quieter, hyperkineses decreased, began to use complex sentences in his speech, self-service skills appeared, i.e., took meals aidless. An improvement in optical functions included the following: the boy began to speak that saw the sun and the sky, saw the outlines of objects, named some objects in pictures.

### Example 10. Cerebral palsy.

A 3.5-year-old patient with diagnosis of cerebral palsy, spastic tetraparesis, symptomatic focal epilepsy, retarded psycholinguistic development was in a stage of medication remission for 1.5 years.

In the absence of speech, the child had the only ability to reproduce separate vowel sounds, his articulation was severely destroyed. Almost always his eyes were closed by lids. A manifest paresis was observed primarily in the lower extremities, the ability to ambulate aidless was absent. The patient hardly maintained a vertical posture even leaning on objects and feebly grasped objects. The degree of spasticity in the upper extremity was from 2 to 3 points, the degree of foot plasticity in the lower extremity equaled to 3 points.

Taking into consideration complaints, clinical signs and symptoms of disease, a progradient course of disease and the failure of the preceding therapy, twofold intravenous infusions of umbilical cord blood cells in a total dose of 500 mln cells (about 2.4x10⁸ and 2.6x10⁸ per infusion, respectively) were administered to the child with a 14-day interval.

Six months post-infusion, a pronounced improvement of clinical signs and symptoms was observed. The child began to fix his glance on surrounding persons, overcame the retardation in speech development, his impressive speech began to conform to his age, he pointed to pictures of all topical groups. The restoration of the articulatory apparatus was noted, namely, switching and maintenance of the posture improved, he grabbled, walked with support or leaning on objects, grasped and held toys, pencils. The spasticity degree decreased by 1 point, muscular strength of the extremities increased by 1 point.

Perception of colors and subjects began to conform to his age. Quantitative notions and the ability to operate with comparisons and generalizations began to form. The patient began to understand the speech in full scope and to construct phrases.

Reference Example 11. Multiple sclerosis.

A male patient, 37 years old, had multiple sclerosis with a primary-progradient course and 8-year duration of disease. His neurological status included a pronounced lower spastic paresis with muscular strength in the lower extremities equaling 2 points, pronounced cerebellar syndrome, pelvic disorders.

The level of disorders equaled to 6.5 points by EDSS. The level of cognitive disorders corresponded to 30 and 32 points by MMSE and PASAT, respectively. The MR spectroscopy findings: NAA\Cr=1.62 ± 0.34; Cho\Cr= 1.09± 0.17.

Twofold infusions of blood-group and rhesus-factor compatible umbilical cord blood cells were administered to the patient in the quantity of 2.5x10⁸ cells per infusion (the total dose of infused cells amounted to about 5x10⁸) with a two-week interval.

After the second infusion and within 1 month, an amelioration in intensity of pyramidal syndrome was observed with lesser time of passing through 7.2 m (45 sec prior to and 40 sec after infusion). The EDSS level remained as before. MMSE and PASAT scores equaled to 30 and 41, respectively. The MR spectroscopy findings: NAA\Cr=1.66 ± 0.25; Cho\Cr= 0.96± 0.17. Thus, a tendency towards an increased level of NAA marker was observed in respect to brain metabolites, which is indicative of an improved state of neurons.

### Example 12. Treatment of posttraumatic encephalopathy in patients that suffered severe traumatic brain injury.

Using the umbilical cells according to the claimed invention was studied on patients that suffered severe traumatic brain injury. Their age varied from 21 to 50 years. The posttraumatic period ranged from 3 to 5 months. The patients demonstrated stable significant asthenic symptoms with a distinct adynamic component, emotional and sensor hyperesthesia, local motor disorders, disordered sensitivity of varied kinds, focal cortical disorders such as aphasia, apraxia, alexia, etc.

Three patients suffered severe brain contusion with cerebral compression and intracranial hematoma. Craniotomy and hematoma removal were performed on these patients. According to computer tomography images 2 other patients had contusion foci in temporal regions and subarachnoidal hemorrhages.

All patients were in a compensated state, their psychoneurological status showed an asthenic syndrome in a varied degree of manifestation. Two patients had moderately manifest left-sided hemiparesis, speech disorders 5 months after injury.

Patients received single or double drop-by-drop intravenous infusion of washed from cryoprotectant, blood group and rhesus factor compatible umbilical cord blood cells preferably from about 2,2x10⁸ to about 2,8x10⁸ of nucleated cells per infusion, in an average amounting to about 2.5x10⁸ cells per infusion. Administration of the concentration of umbilical cord blood stem cells was not accompanied by any reactions on the part of cardiovascular and respiratory systems, only one patient had transitory temperature rise to subfebrile values.

After administration of cells all patients demonstrated a distinct tendency to rapid (within three months) reduction of all structural components of asthenic syndrome: increased fatigability, cachexia, affective lability, hyperesthesia, phenomena of vegetative dysfunction, drowsiness. The level of initiative and mental activity increased essentially.

Three months after administration of umbilical cord blood cells, two patients with paresis demonstrated its practically complete regression. The patient with aphasic disorders demonstrated essential amelioration of speech functions: elements of motor aphasia regressed and speech rate accelerated.

The tempo and successful performance of functional and neuropsychological tasks which were in one or another way connected with the parietal-occipital activity irrespective of real localization of brain damage have increased. Thus, the patients began to solve the tasks of spatial organization of movements easier, began to solve arithmetic tasks better, etc. A revealed tendency towards the lower values of asthenia, anxiety and somatic symptoms of anxiety demonstrates an ameliorated psychological wellbeing of patients two of which returned to labor activity.

Thus, the patients of the study group have demonstrated evident amelioration of their state. The data obtained have suggested that therapy using umbilical cord blood cells produces antiasthenic and nootropic effects accompanied by accelerated restoration of cognitive and "instrumental" cortical functions.

### Example 13. Treatment of posttraumatic encephalopathy with a pronounced asthenic syndrome, speech disorders and focal neurological symptoms.

Patient P., aged 21 years, received head trauma as a result of a traffic accident. He was admitted to the neurosurgical unit of a municipal hospital as an emergency case. Within 2 weeks the patient was in a severe condition in a resuscitation unit: deranged consciousness up to coma I, deep right-sided paresis was formed. Thereafter, he was admitted to the inpatient hospital with the diagnosis "Severe brain contusion, contused wound of the right parieto-temporal region". At the background of the vascular nootropic infusion therapy administered, his state gradually ameliorated, consciousness returned. However, for a long time, severe speech disorders (in an acute period within a month he could not speak at all, instead of articulated speech he could only mumble), pronounced right-sided hemiparesis, weakness and fatigability persisted. Later on, the patient received an outpatient treatment, and neurological symptomatology regressed only partially. Nonetheless, the patient came back to labor, but had difficulties in performing even easy work task, got tired rapidly. He complained of speech disorders, lowered memory, retarded mentality, clumsiness and motor weakness in the left extremities, fatigability.

His psychoneurological status showed moderately pronounced speech and mnestic disorders, distinct right-sided hemiparesis more pronounced in the upper extremity. Neuropsychological examination revealed disorders in higher cortical functions manifested primarily in cognitive disorders by the right-hemisphere type.

Computer tomography did not reveal the signs of cerebral pathology. In the examination of glucose metabolism a series of positron-emission tomograms revealed focal and diffuse changes in the right hemisphere. A slightly decreased glucose metabolism (up to 13% versus the contralateral area) was revealed in the pole of the right temporal lobe. In parasagittal areas of major hemispheres at the juncture of frontal and parietal lobes, the level of retention of the preparation was 20% lower as compared with analogous compartments of frontal lobes, without asymmetry. Diffuse glucose metabolism was observed in the right thalamus. Findings of electroencephalography (EEG) did not reveal focal pathological typical epileptiform activity.

The results of duplex investigation bear evidence of a slightly decelerated blood flow along the right posterior cerebral artery, moderate disarrangement of the vascular tone and cerebrovascular reactivity.

The patient received treatment using the claimed invention. Umbilical cord blood cells were transplanted in the quantity of 2.6x10⁸ by single intravenous infusion. The procedure was well tolerated, no adverse effects were observed.

Within three months post-infusion, practically complete regression of paresis phenomena in the right extremities, improved speech functions (speech rate increased, it became more fluent) was observed. The patient came back to usual rhythm of his labor activity, asthenic phenomena disappeared completely.

According to results of psychological investigation, the patient's long-term memory capacity has increased, the level of fatigability has decreased and the speed of task performance has accelerated.

No changes in bioelectric activity of the brain were revealed within the follow-up period.

Compared to the results of the previous duplex investigation, positive dynamics was observed in terms of normalized peripheral resistance (resistance indices) in the cerebral basement arteries and accelerated blood flow along the cerebral basement arteries.

Changes in clinical and biochemical blood counts, immune status were within normal limits.

Thus, the patient had an observable improvement of his state. The findings obtained suggest that therapy using umbilical cord blood stem cells produces antiasthenic and nootropic effects accompanied by accelerated restoration of cognitive and "instrumental" cortical functions.

### Example 14. Treatment of posttraumatic encephalopathy with a moderately pronounced asthenic syndrome.

Patient A., aged 40 years, received trauma as a result of a blow to the head.

A short-term blackout was noted, afterwards, within some days he suffered nausea, vomiting, and gradually increased intensive headaches. Magneto-resonance tomography revealed subdural hematoma on the right.

Craniotomy and hematoma removal were performed on the patient. The postoperative course was uneventful. At the background of the vascular antibacterial nootropic infusion therapy administered, his state has gradually ameliorated. He was discharged in a compensated state to receive out-patient treatment, standard medication therapy was administered, but neurological symptoms persisted.

Changes in the neurological status on admission were presented primarily by moderately manifest asthenic disorders. The patient complained of periodic headaches, pronounced fatigability, lowered labor performance, the presence of a trepanation defect in the right frontal-parietal-temporal region.

A series of computer tomograms did not reveal any signs of cerebral pathology, a trepanation bone defect, 7.5 cm in diameter, was observed in the right parieto-temporal region through which the cerebral matter prolapsed insignificantly (up to 5 mm).

The duplex investigation demonstrated normal length and diameter of extracranial arteries, blood flow was sufficient. When glucose metabolism was studied, a series of tomograms (15 cuts) of the brain revealed a tiny focus of glucose hypometabolism (lowered to 21 %) in the convexital cortex of the right parietal lobe with 6 mm in diameter corresponding to intracerebral hematoma identified by MRT. Around this focus in the cortex of the right parietal lobe, a zone of diffuse decline of the glucose consumption level by 16% is recorded (versus contralateral area). In the convexital cortex of the posterior right temporal lobe, glucose hypometabolism equals to 14%. The decline in retention of the preparation in the cerebellum versus the frontal lobe also equals to 14% (with account of physiological variability). In the remainder cortical compartments of major hemispheres and subcortical structures, no changes of glucose metabolism were revealed. Diagnosis: Traumatic disease of the brain, disordered energy metabolism in the convexital cortex of parietal and posterior temporal lobes of the right major hemisphere, and in the cerebellum as well.

When treating by using the claimed invention, umbilical cord blood cells were transplanted in the quantity of 2.56x10⁸ by single intravenous infusion. The procedure was well tolerated, no adverse effects were observed.

Within three months after infusion, the patient's state has improved essentially, namely, asthenisation phenomena significantly ameliorated, headaches ceased practically completely, the patient began to make plans for his future life, is going to come back to labor activity.

According to results of psychological investigation, the patient's short- and long-term memory capacity has retained to normal limits, the number of mistakes and time for performance of tasks have decreased.

No changes in bioelectric activity of the brain were revealed within the follow-up period as well.

The findings obtained suggest that therapy using umbilical cord blood stem cells produces antiasthenic and nootropic effects accompanied by accelerated restoration of cognitive and "instrumental" cortical functions.

### Example 15. Treatment of posttraumatic encephalopathy with a pronounced asthenic syndrome, personality changes by type of the frontal lobe amotivational syndrome.

Patient T., aged 47 years, with head trauma was admitted to the hospital in a moderate severity state with complaints of heavy headaches and impairment of consciousness. Computer tomography has revealed intracranial hematoma in the right temporal lobe, subarachnoidal hemorrhage, lamellar subdural hematoma. After cerebral trepanation and hematoma removal, at the background of the administered vascular, antibacterial nootropic infusion therapy, his state has ameliorated a little, but without an essential effect. The patient had pronounced asthenic disorders, apathy, atony, rapid fatigability, depressed mood, lowered appetite, insomnia, weight loss, and spent most part of his time in bed.

Computer tomography revealed the signs of dyscirculatory encephalopathy and cicatrical-atrophic changes in the right temporal lobe.

According to results of duplex investigation, there was an atherosclerotic lesion to brachiocephal arteries. The signs of local hemodynamic disorders were revealed in the basin of the major artery as well as echographic signs of the systemic disorder of cerebral hemodynamics in terms of lowered cerebrovascular reactivity with a deficit of vasodilation reaction and slightly impaired venous outflow from the cranial cavity along jugular veins and vertebral plexuses.

Umbilical cord blood cells were transplanted to the patient according to the claimed method in the quantity of 2.73x10⁸ and 2.53x10⁸ by way of intravenous infusion, two times with an interval of 3 weeks. The total quantity of transplanted cells equaled to 5.26x10⁸ No side effects were observed.

The follow-up examination conducted three months later has revealed complete rehabilitation of mental functions, no asthenia and signs of intellectual-mnestic disorders.

The comparison with results of duplex investigation of vessels conducted prior to the treatment by the claimed method has revealed a slightly increased vasodilation reserve. Clinical, immunological and biochemical blood counts were within normal limits.

According to results of psychological investigation, after double infusion of umbilical cord blood cells, a distinct tendency was observed to accelerated rate of the patient's mental activity, an essentially increased effectiveness of modality-nonspecific properties of memory and attention (including lowered cachexia, improvement in selectivity, concentration and switchover capacity).

Results of EEC assessment of the electrophysiological state of the patient's brain with investigation of cognitive potentials have demonstrated that, the medium frequency of alpha fluctuations shifted to the high-frequency region after the first infusion of umbilical cord blood cells, and the oscillation amplitude decreased after the repeated infusion.

The results of investigation of energy metabolism of the patient's brain using positron-emission tomography have revealed that glucose metabolism decreased by 9-3% in the convexital cortex of the right parietal lobe where glucose hypometabolism (14%-7%) was identified at the primary examination. Besides, a distinct decrease in the functional thinning of the cortex was observed.

Thus, as a result of the treatment administered according to the claimed invention, significant restoration of mental functions was observed in the patient. Positive clinical dynamics was accompanied by positive dynamics of electrophysiological and metabolic processes in the brain.

## Claims

1. Umbilical cord blood cells for use in a method of treatment of a human patient who is suffering from degenerative changes in the brain selected from posttraumatic encephalopathy and cerebral palsy.

2. The cells for use according to claim 1, wherein the amount of cells administered to a patient in said method ranges from 1x10⁸ to 5x10⁸ nucleated cells per infusion.

3. The cells for use according to claim 2, wherein the amount of cells ranges from 2.2x10⁸ to 2.8x10⁸ nucleated cells per infusion.

4. The cells for use according to any one of the preceding claims, wherein umbilical cord blood nucleated cells are administered repeatedly to a patient in said method with a 1-4-week interval.

5. The cells for use according to any one of the preceding claims, wherein umbilical cord blood nucleated cells are administered up to four times to a patient in said method.

6. Use of umbilical cord blood cells for the manufacture of a medicament for use in a method of treatment of a human patient who is suffering from degenerative changes in the brain selected from posttraumatic encephalopathy and cerebral palsy.

7. Use according to claim 6, wherein the amount of cells administered to a patient in said method ranges from 1x10⁸ to 5x10⁸ nucleated cells per infusion.

8. Use according to claim 7, wherein the amount of cells ranges from 2.2x10⁸ to 2.8x10⁸ nucleated cells per infusion.

9. Use according to any one of claims 6 to 8, wherein umbilical cord blood nucleated cells are administered repeatedly to a patient in said method with a 1-4-week interval.

10. Use according to any one of claims 6 to 9, wherein umbilical cord blood nucleated cells are administered up to four times to a patient in said method.

## Patentansprüche

1. Nabelschnurblutzellen zur Verwendung in einem Verfahren zur Behandlung eines menschlichen Patienten, der an degenerativen Veränderungen im Gehirn, ausgewählt aus posttraumatischer Enzephalopathie und Zerebralpalsy, leidet.

2. Zellen zur Verwendung gemäß Anspruch 1, wobei die Menge an Zellen, die einem Patienten in dem Verfahren verabreicht wird, von 1x10⁸ bis 5x10⁸ kernhaltige Zellen pro Infusion reicht.

3. Zellen zur Verwendung gemäß Anspruch 2, wobei die Menge an Zellen von 2,2x10⁸ bis 2,8x10⁸ kernhaltige Zellen pro Infusion reicht.

4. Zellen zur Verwendung gemäß einem der vorangehenden Ansprüche, wobei kernhaltige Nabelschnurblutzellen wiederholt an einen Patienten in dem Verfahren mit einem 1-4-Wochen-Intervall verabreicht werden.

5. Zellen zur Verwendung gemäß einem der vorangehenden Ansprüche, wobei kernhaltige Nabelschnurblutzellen in dem Verfahren bis zu viermal an einen Patienten verabreicht werden.

6. Verwendung von Nabelschnurblutzellen für die Herstellung eines Medikaments zur Verwendung in einem Verfahren zur Behandlung eines menschlichen Patienten, der an degenerativen Veränderungen im Gehirn, ausgewählt aus posttraumatischer Enzephalopathie und Zerebralpalsy, leidet.

7. Verwendung gemäß Anspruch 6, wobei die Menge an Zellen, die einem Patienten in dem Verfahren verabreicht wird, von 1x10⁸ bis 5x10⁸ kernhaltige Zellen pro Infusion reicht.

8. Verwendung gemäß Anspruch 7, wobei die Mengen an Zellen von 2,2x10⁸ bis 2,8x10⁸ kernhaltige Zellen pro Infusion reicht.

9. Verwendung gemäß einem der Ansprüche 6 bis 8, wobei kernhaltige Nabelschnurblutzellen wiederholt in dem Verfahren mit einem 1-4-Wochen-Intervall an einen Patienten verabreicht werden.

10. Verwendung gemäß einem der Ansprüche 6 bis 9, wobei kernhaltige Nabelschnurblutzellen in dem Verfahren bis zu viermal an einen Patienten verabreicht werden.

## Revendications

1. Cellules de sang de cordon ombilical pour utilisation dans un procédé de traitement d'un patient humain souffrant d'altérations dégénératives dans le cerveau, choisies parmi une encéphalopathie post-traumatique et une infirmité motrice cérébrale.

2. Cellules pour utilisation conformes à la revendication 1, la quantité de cellules administrée au patient dans ledit procédé valant de 1.10⁸ à 5.10⁸ cellules nucléées par injection.

3. Cellules pour utilisation conformes à la revendication 2, la quantité de cellules valant de 2,2.10⁸ à 2,8.10⁸ cellules nucléées par injection.

4. Cellules pour utilisation conformes à l'une des revendications précédentes, des cellules nucléées de sang de cordon ombilical étant, dans ledit procédé, administrées de façon répétée à un patient à intervalles de 1 à 4 semaines.

5. Cellules pour utilisation conformes à l'une des revendications précédentes, des cellules nucléées de sang de cordon ombilical étant, dans ledit procédé, administrées jusqu'à quatre fois à un patient.

6. Utilisation de cellules de sang de cordon ombilical pour la fabrication d'un médicament destiné à être utilisé dans un procédé de traitement d'un patient humain souffrant d'altérations dégénératives dans le cerveau, choisies parmi une encéphalopathie post-traumatique et une infirmité motrice cérébrale.

7. Utilisation conforme à la revendication 6, dans laquelle la quantité de cellules administrée au patient dans ledit procédé vaut de 1.10⁸ à 5.10⁸ cellules nucléées par injection.

8. Utilisation conforme à la revendication 7, dans laquelle la quantité de cellules vaut de 2,2.10⁸ à 2,8.10⁸ cellules nucléées par injection.

9. Utilisation conforme à l'une des revendications 6 à 8, dans laquelle des cellules nucléées de sang de cordon ombilical sont, dans ledit procédé, administrées de façon répétée à un patient à intervalles de 1 à 4 semaines.

10. Utilisation conforme à l'une des revendications 6 à 9, dans laquelle des cellules nucléées de sang de cordon ombilical sont, dans ledit procédé, administrées jusqu'à quatre fois à un patient.
